# EUROPEAN PATENT SPECIFICATION

(11) **EP 4 291 126 B1**
(45) Date of publication and mention of the grant of the patent: **15.10.2025**
(21) Application number: 22707290.7
(22) Date of filing: 09.02.2022
(51) Int. Cl.: A61F 2/30, A61F 2/38, A61B 34/00, A61B 34/10, A61B 34/20

(54) **SYSTEMS FOR PLANNING AND PERFORMING IMPLANT SURGERY**
SYSTEME ZUR PLANUNG UND DURCHFÜHRUNG VON IMPLANTATCHIRURGIE
SYSTÈMES DE PLANIFICATION ET DE RÉALISATION D'UNE CHIRURGIE D'IMPLANT

(30) Priority: 11.02.2021 US 202163148486 P
(43) Date of publication of application: 20.12.2023
(73) Proprietor: Smith & Nephew, Inc., Memphis. Tennessee 38116 (US); Smith & Nephew Orthopaedics AG, 6300 Zug (CH); Smith & Nephew Asia Pacific Pte. Limited, Singapore 138565 (SG)
(72) Inventor: NIKOU, Constantinos, Pittsburgh, PA 15222 (US); JARAMAZ, Branislav, Pittsburgh, Pennsylvania 15222 (US); DUMPE, Samuel C., Pittsburgh, Pennsylvania 15222 (US); KHARE, Rahul, Pittsburgh, Pennsylvania 15222 (US)
(74) Representative: Appleyard Lees IP LLP
(86) International application number: PCT/US2022/015726
(87) International publication number: WO 2022/173775

(56) References cited:
- EP-B1- 2 373 243
- US-A1- 2014 019 110
- US-A1- 2020 205 898

## Description

### CROSS-REFERENCE TO RELATED APPLICATION

This application claims the benefit of U.S. Provisional Patent Application Serial No. 63/148,486, filed February 11, 2021 and titled "Methods and Systems for Planning and Performing Implant surgery".

### FIELD OF THE DISCLOSURE

The present disclosure is directed to methods and systems for computer-assisted surgical systems, and more particularly to techniques for model-based surgical planning.

### BACKGROUND

Arthroplasty procedures, such as total knee arthroplasty (TKA), may use a surgical plan to define, among other things, accommodations (e.g., resections, reaming, etc.) to patient bone to accept an implant and an implant position target. The position and orientation of the implant may be determined in the surgical plan prior to performing the surgery. However, a surgeon may often modify the plan during the operation based on information obtained as the procedure progresses, for example, regarding the condition of the patient bone, implant fit, and/or the like.

Various existing computer-assisted systems may be used to generate and/or modify a surgical plan. Conventional computer-assisted systems typically require the surgeon to simultaneously evaluate the effect of numerous parameters, such as implant angles and distances, and implant placement during the creation or modification of the surgical plan. In addition, a change in one parameter may have multiple consequences on other aspects of implant fit and performance. It is challenging for the surgeon to effectively evaluate each potential consequence of a change to the surgical plan, particularly in a time frame required during a live operation. Accordingly, a surgeon using a conventional system may have difficulty efficiently determining the optimal placement of an implant.

It is with this in mind that the present disclosure is provided. EP 2373243 B1 discloses implant planning for multiple implant components using constraints. US 2014/0019110 A1 discloses operatively tuning implants for increased performance. US 2020/0205898 A1 discloses methods for surgical planning using soft tissue attachment points.

### SUMMARY

The present invention concerns a surgical system comprising the features defined in the independent claim 1. Preferred embodiments are defined in the dependent claims. This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

The present disclosure relates generally to methods and apparatuses related to a computer-assisted surgical system that includes various hardware and software components that work together to enhance surgical workflows. In various embodiments, the computer-assisted surgical system may operate to perform or facilitate the performance of an implant surgery that may include an arthroplasty procedure, including, for example and without limitation, a total knee arthroplasty (TKA), a revision TKA (rTKA), a total hip arthroplasty (THA), and/or the like.

In some embodiments, methods, systems, apparatuses, devices, and/or combinations thereof may provide a surgical planning process for generating a surgical plan for the implant surgery. The surgical planning process may include accessing an anatomical model of a portion of the patient to be fitted with the implant. For example, for a TKA or rTKA procedure, the anatomical model may include a model of an end portion of a tibia configured to receive a tibial tray implant device (for instance, the proximal tibia). The anatomical model may include at least one interface region configured to couple with or otherwise receive a corresponding interface element of the implant. For example, a tibia anatomical model may include an intramedullary (IM) canal interface region configured to receive a corresponding stem interface element of a tibial tray.

A constraint model may be generated that is configured to model valid areas of placement of the interface element within the interface region. For example, the constraint model for the tibia anatomical model may define a constraint space for placement of the stem of the tibial tray within the IM canal. In some embodiments, the fit provided by the constraint model may restrict implant placement and further limit how implants may be positioned or repositioned relative to any valid placement. For example, placement of an implant may be limited to only valid positions and/or orientations that do not violate or are within a violation threshold of the area defined by the constraint space.

In one embodiment, a method for determining a surgical plan may include determining a constraint model associated with an anatomical model. The constraint model may be configured to indicate at least one valid configuration of an implant model in association with the anatomical model.

In one embodiment, a method for determining a surgical plan may include accessing an anatomical model comprising at least one model interface for engaging an implant interface of an implant model, determining a constraint model associated with the model interface, the constraint model may be configured to indicate at least one valid configuration of the implant interface in association with the model interface.

In one embodiment, a method for determining a surgical plan may include determining a constraint model associated with an anatomical model, the constraint model configured to indicate at least one valid configuration of an implant model in association with the anatomical model, receiving placement input indicating placement of the implant in association with the anatomical model, and performing, based on the placement input, a violation process responsive to determining a violation event, or a placement process positioning the implant according to the placement input.

In one embodiment, an apparatus may include at least one processor and a memory coupled to the at least one processor. The memory may include instructions that, when executed by the at least one processor, may cause the at least one processor to determine a constraint model associated with an anatomical model the constraint model configured to indicate at least one valid configuration of an implant model in association with the anatomical model, receive placement input indicating placement of the implant in association with the anatomical model, and performing, based on the placement input, a violation process responsive to determining a violation event, or a placement process positioning the implant according to the placement input.

In one embodiment, the surgical plan may be configured for an arthroplasty surgical procedure. In one embodiment, the arthroplasty surgical procedure may include a TKA or an rTKA procedure.

In one embodiment, the anatomical model may include at least a portion of a femur or at least a portion of a tibia. In one embodiment, the anatomical model may include at least a portion of a distal femur configured to receive a femoral component knee implant device. In one embodiment, the anatomical model may include at least a portion of a proximal tibia configured to receive a tibial knee implant device. In one embodiment, a model interface may include an IM canal. In one embodiment, an implant interface may include an IM stem configured to engage the IM canal. In one embodiment, the constraint model may include a constraint space defined by a volume of the IM canal.

One embodiment may include presenting at least one indicator indicating a violation responsive to a violation event. One embodiment may include presenting at least one indicator indicating that the implant is not violating the constraint model. In one embodiment, the at least one indicator may include at least one of an overlay, a color, a pattern, an alert, an alarm, a message, or a tactile signal.

In one embodiment, a violation event process may operate to handle input that violates the constraint model. In one embodiment, a violation event process may prevent user input that violates the constraint model by preventing motion input moving the implant into a violating position or setting a parameter that causes a violating position. In one embodiment, a violation event process may prevent input by limiting parameter values that violate the constraint model.

In one embodiment, movement of the anatomical model may be bound within a region defined by the constraint model to eliminate invalid model configurations.

In one embodiment, the anatomical model may be presented via a user interface screen. In one embodiment, the user interface screen may include input objects for receiving user input to manipulate the implant model. In one embodiment, the input objects may include functions to move the implant model. In one embodiment, at least one function may include a clockface dial to move a radial offset of a portion of the implant model. In one embodiment, the user interface screen may present performance information associated with a displayed configuration of the implant model. In one embodiment, the performance information is updated responsive to a change in the configuration of the implant model.

In one embodiment, a surgical plan may be determined based on a configuration of the implant model within the constraint model. In one embodiment, a surgical procedure may be administered based on the surgical plan.

A surgical system includes at least one computing device. The at least one computing device includes processing circuitry and a memory coupled to the processing circuitry. The memory includes instructions that, when executed by the processing circuitry, causes the processing circuitry to determine a constraint model associated with an anatomical model, the constraint model configured to model at least one valid configuration of an implant model in association with the anatomical model, wherein the anatomical model comprises at least one model interface for engaging an implant interface of the implant model, the constraint model configured to indicate at least one valid configuration of the implant interface in association with the model interface, wherein the model interface comprises an intramedullary (IM) canal interface, the implant interface comprising an IM stem configured to engage the IM canal, wherein the constraint model comprises a constraint space defined, at least partially, by a volume of the IM canal, receive placement input indicating placement of the implant in association with the anatomical model, and perform, based on the placement input one of a violation process based on a violation event responsive to the placement input violating the constraint model, the violation process operative to prevent placement input moving the implant into a position that violates the constraint model, or a placement process positioning the implant according to the placement input responsive to the placement input not violating the constraint model.

In one embodiment of the surgical system, the instructions, when executed by the processing circuitry, may cause the processing circuitry to generate at least one indicator indicating a violation responsive to the violation event. In one embodiment of the surgical system, the at least one indicator may include at least one of an overlay, a color, a pattern, an alert, an alarm, a message, or a tactile signal.

In one embodiment of the surgical system, the violation event process may operate to prevent user input that violates the constraint model via limiting parameter values that violate the constraint model. In one embodiment of the surgical system, wherein movement of the model may be bound within a region defined by the constraint model to prevent model configurations that violate the constraint model.

In one embodiment of the surgical system, the instructions, when executed by the processing circuitry, may cause the processing circuitry to generate a surgical plan determined based on a configuration of the implant model within the constraint model.

In one embodiment of the surgical system, the constraint model may be configured for an arthroplasty surgical procedure. In one embodiment of the surgical system, the anatomical model may include at least a portion of a distal femur configured to receive a femoral component knee implant device. In one embodiment of the surgical system, the anatomical model may include at least a portion of a proximal tibia configured to receive a tibial knee implant device.

In one embodiment of the surgical system, the at least one valid configuration may be based on at least one implant placement condition comprising at least one of an anatomical fit or a performance criterion of an implant.

In one embodiment of the surgical system, the constraint model may be configured to model at least one degree-of-freedom of an implant, the at least one degree-of-freedom comprising at least one of a varus/valgus angle, a flexion/extension position, a medial/lateral position, or an anterior/posterior position

In one embodiment of the surgical system, the constraint model may be generated to correspond to surgically-prepared patient anatomy.

Embodiments of the present disclosure provide numerous technological advantages and technical features over conventional systems. One non-limiting example of a technological advantage may include providing a surgical planning process using constraint models configured to model valid positions of an implant on or within patient anatomy. Surgical planning processes according to some embodiments may, among other things, facilitate planning of implant device positioning with decreased complexity and improved efficiency and effectiveness as compared to conventional systems. For example, surgical planning processes according to some embodiments may allow healthcare professionals to avoid evaluation of invalid placement configurations during the planning of a surgical procedure, saving time and effort. A further non-limiting example of a technological advantage may include providing improved arthroplasty procedure planning processes using constraint modeling. An additional non-limiting example of a technological advantage may include providing improved user interfaces and workflows for planning rTKA procedures, which have not been adequately addressed by conventional systems. Embodiments are not limited in this context. Additional technological advantages and technical features over conventional systems would be known to those of skill in the art based on the present disclosure.

Embodiments described in the present disclosure may provide improvements in technology, including, for example, computing technology, and/or a technical field. For example, managing the complexity of planning the placement of an implant device in or on patient anatomy has long been problematic for healthcare professionals using conventional computer-assisted tools, particularly managing changes during a surgical procedure. The placement of an implant device involves multiple parameters (e.g., varus/valgus angle, flexion/extension angles and/or offsets, and/or the like). These parameters lead to a multidimensionally complex configuration space which the surgeon (or other healthcare professional) has to explore while trying to achieve the goals of the surgery, for instance, retaining or regaining proper kinematics of a knee j oint. A change in the implant position and/or parameters may have a cascading effect on multiple other properties, that may not be noticeable to a surgeon.

Computer-assisted surgical planning processes according to some embodiments may provide improvements in computing technology and/or the technical field of surgery (or surgical systems, surgical navigation systems, and/or the like) through, for example and without limitation, determining a constraint model of valid positions and/or parameter values (or valid positions and/or parameter values within a threshold) to facilitate easier and more efficient evaluation of implant placement by limiting the scope of evaluation for the surgeon. Surgical planning processes according to some embodiments may allow the surgeon to focus on configuring valid implant placement options, while avoiding spending time, effort, and resources on potentially invalid implant placement options. Embodiments are not limited in this context. Additional improvements to computing technology would be known to those of skill in the art based on the present disclosure.

Surgical planning processes according to some embodiments may be applied to various practical applications. For example, constraint models may be applied by a surgeon and/or computing system to generate a surgical plan. In another example, constraint models may be applied by a surgeon and/or computing system to administer surgery to a patient, such as performing an arthroplasty procedure. In an additional example, surgical planning processes (and constraint models thereof) may be integrated into a surgical system hardware and/or hardware used to plan and/or perform a surgical procedure. Embodiments are not limited in this context. Additional practical applications would be known to those of skill in the art based on the present disclosure.

Further features and advantages of at least some of the embodiments of the present invention, as well as the structure and operation of various embodiments of the present invention, are described in detail below with reference to the accompanying drawings.

### BRIEF DESCRIPTION OF THE DRAWINGS

By way of example, a specific embodiment of the disclosed methods, systems, and apparatuses will now be described, with reference to the accompanying drawings, in which:
**FIG. 1** is a diagram illustrating an environment for operating a system for planning and performing an arthroplasty surgery in accordance with one or more features of the present disclosure;
**FIG. 2** is a block diagram depicting a system for performing a surgery planning process in accordance with one or more features of the present disclosure;
**FIGS. 3A-3C** depicts an illustrative graphical user interface for performing a surgery planning process in accordance with one or more features of the present disclosure;
**FIG. 4** depicts an illustrative graphical user interface for performing a surgery planning process in accordance with one or more features of the present disclosure;
**FIG. 5A** is an exploded, perspective view of an example of an embodiment of a knee prosthesis (e.g., a femoral component or implant) in accordance with one feature of the present disclosure;
**FIG. 5B** is an exploded, perspective view of an example of an embodiment of a knee prosthesis (e.g., a tibial component or implant) in accordance with one feature of the present disclosure;
**FIG. 6** depicts an illustrative graphical user interface for performing a surgery planning process in accordance with one or more features of the present disclosure;
**FIG. 7** illustrates a first logic flow in accordance with the present disclosure;
**FIG. 8** illustrates a second logic flow in accordance with the present disclosure; and
**FIG. 9** illustrates an embodiment of a computing architecture in accordance with the present disclosure.

The drawings are not necessarily to scale. The drawings are merely representations, not intended to portray specific parameters of the disclosure. The drawings are intended to depict example embodiments of the disclosure, and therefore are not to be considered as limiting in scope. In the drawings, like numbering represents like elements.

Furthermore, certain elements in some of the figures may be omitted, or illustrated not-to-scale, for illustrative clarity. The cross-sectional views may be in the form of "slices", or "near-sighted" cross-sectional views, omitting certain background lines otherwise visible in a "true" cross-sectional view, for illustrative clarity. Furthermore, for clarity, some reference numbers may be omitted in certain drawings.

### DETAILED DESCRIPTION

Various features or the like of methods, systems, and/or apparatuses of a surgical planning process will now be described more fully hereinafter with reference to the accompanying drawings, in which one or more features of the surgical planning process will be shown and described. In various embodiments, for example, the surgical planning process may be used as part of a computer-assisted arthroplasty procedure, for instance, implemented using a surgical navigation system. It should be appreciated that the various features may be used independently of, or in combination, with each other. It will be appreciated that the surgical planning process as disclosed herein may be embodied in many different forms and should not be construed as being limited to the embodiments set forth herein. Rather, these embodiments are provided so that this disclosure will convey certain features of the surgical planning process to those skilled in the art.

As will be described herein, in accordance with one or more features of the present disclosure, a computer-assisted surgical planning process arranged and configured for use in planning and/or performing a surgical procedure is disclosed. In some embodiments, the surgical planning process may be configured for use in an arthroplasty procedure, for example, for planning the placement of an implant device on and/or within patient anatomy. For example, the surgical planning process may include a method of determining the placement of a femoral component of a knee prosthetic device within the femur of a patient. In another example, the surgical planning process may include a method of determining the placement of a tibial component of a knee prosthetic device within the tibia of a patient. In various embodiments, the surgical planning process may be or may include a revision total knee arthroplasty (rTKA) procedure.

Although knee prosthetic devices, knee arthroplasty procedures (including rTKA procedures), femoral components of knee prosthetic devices, and tibial components of knee prosthetic devices are used in the present disclosure, embodiments are not so limited as these examples are provided for illustrative purposes. A person of skill in the art would recognize that the described embodiments may be applied to various other types of surgeries, prosthetic devices, anatomical regions, and/or the like.

In one embodiment, as will be appreciated by one of ordinary skill, the surgical planning process may operate, at least in part, using constraint modeling. In some embodiments, constraint modeling may be or may include modeling (or otherwise determining) valid and/or invalid placement of an implant in an anatomical model. In some embodiments, implant placement may refer to a location of the implant with respect to an anatomical model (and, indirectly, to the corresponding patient anatomy being represented by the anatomical model) and/or associated parameters (for instance, varus/valgus angle, flexion/extension angles and/or offsets, and/or the like).

Accordingly, in exemplary embodiments, constraint modeling may include defining a constraint space of valid implant placement. In general, valid implant configurations or placements may include implant placements that do not violate (or violate within a threshold or other variance) one or more implant placement conditions, including, without limitation, fitting on or within anatomy (for instance, an implant stem of a tibial component fitting within the intramedullary (IM) canal of the tibia), performance criteria or goals (for instance, placement of the implant may be required to meet certain performance metrics, such as range of motion, gait, deformities, and/or the like), placement criteria or goals (for instance, gap placement criteria, varus/valgus angle criteria, and/or the like), combinations thereof, and/or the like. Accordingly, use of constraint modeling in surgical planning processes according to some embodiments may allow a surgeon to focus on configuring valid (and/or optimal) implant placement options, while avoiding spending time, effort, and resources on potentially invalid (or sub-optimal) implant placement options.

In revision knee replacements (for instance, an rTKA procedure), the typical femoral component and/or tibial component may be supported by an attached IM stem that extends into the IM canal of the femur/tibia. The stem connection means most revision components are typically fixed at a set varus/valgus (V/V) angle coronally (for instance, typically, about six (6) degrees). However, due to variations in bone anatomy, this varus/valgus (V/V) angle can vary from patient to patient. In another embodiment, the variation in bony anatomy may also vary sagittally relative to the articular cartilage geometry (i.e., distal exterior/outside part of the bone) and the position of the IM canal (i.e., interior/inside of the bone). It may be desirable to place the articular part of the femoral component in the most optimal position for a given patient relative to a mechanical or anatomical axis in regards to the V/V angle coronally while optimally positioning the anterior/posterior (A/P) position, medial/lateral (M/L) position, and to a lesser extent, the internal/external rotation (on a transverse plane) while rigidly fixing a stem to the femoral component in a position such that the stem fits centrally within the intermedullary canal.

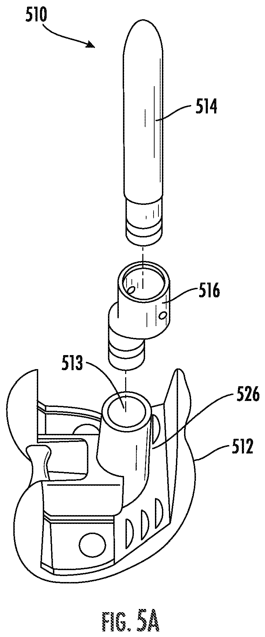

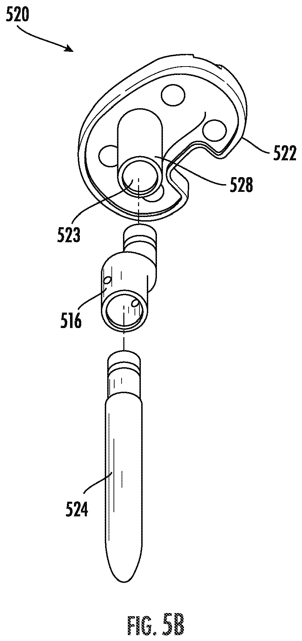

Some embodiments may provide surgical planning processes for femoral and/or tibial components that include an intermediate stem extension or coupler that couples a femoral or tibial articular component (e.g., tibial tray and articular insert) with a stem extension and/or tracking component (see, for example, FIGS. 5A and 5B). Furthermore, the coupler stem connection may be offset from the articular component or tracking component at a known distance and orientation and is angled relative to the articular components varus/valgus and/or flexion/extension (F/E) desired angular orientation and the desired stem's angular orientation. Such femoral or tibial components may allow variable alignment to an anatomical or kinematic alignment or to a surgeon-prescribed alignment such as human bone deformities, muscle structures, and/or flexion/extension balance.

Accordingly, rTKA procedure planning is often constrained by the IM canal, whether natively used or as reamed by the surgeon. This canal constricts the position of proximal aspects of implant stems (or stem extensions, couplers, and/or the like) in both the femur and tibia, in particular the longer stems often required during rTKA. In addition, for example, rTKA planning often involves use of modular offset parameters which locate the distal part of the implant radially from the stem axis. The distance of the offset and the radial angle of the offset are parameters that are chosen by the surgeon to support stem alignment within the canal, while allowing for adjustments of anterior/posterior and medial/lateral adjustment of the distal femur or proximal tibia joint surface, in order to achieve goals of implant centralization, and adjustment of the flexion gaps. Furthermore, rTKA implants could also support a modular varus/valgus or flexion/extension "offset," which allows for setting the tilt of the distal implant surface relative to the implant stem.

Robotic- or computer-assisted knee replacement procedures may include one or more planning steps where the surgeon chooses the location of the implant via a "free placement" interface where the surgeon is tasked with determining the optimal location of the implant. However, such "free placement" user interfaces require the surgeon to evaluate a multitude of possible configurations, locations, parameters and or the like, which is time consuming and prone to error (particularly during a live surgical procedure) (e.g., involving a complex, inefficient trial-and-error approach). In addition, "free placement" user interfaces allow the surgeon to plan the implant position in a place that violates the limitations imposed by the canal (or other anatomical or implant features). The numerous and intricate parameters lead to a multidimensionally complex configuration space which the surgeon has to evaluate while trying to achieve the goals of the surgery, for instance, retaining or regaining proper kinematics of the knee joint.

Accordingly, some embodiments may include methods, systems, and/or apparatuses that access an anatomical model of a portion of patient anatomy being fitted for an implant. For example, an anatomical model may include a three-dimensional (3D) model of a femur or tibia, which may include a model or map of the IM canal. A constraint space or model may be generated based on one or more anatomical interfaces of the patient anatomy and/or implant interfaces of the implant. For example, an anatomical interface may include an IM canal and an implant interface may include a stem of an implant configured to be fitted into the IM canal. In another example, an anatomical interface may include a resected portion of a tibia configured to engage an implant interface in the form of a tibial tray of a tibial implant component.

In some embodiments, the constraint model may operate to model valid placement positions, parameters, and/or the like of the implant interface on or within the anatomical interface. For example, a constraint model may include an anatomical interface of an IM canal (or a portion of an IM canal estimated to interface with a corresponding portion of the implant). In another example, an implant interface may include an IM stem (or extension, coupler, and/or the like) configured to be installed within the IM canal of a femur or tibia. Accordingly, for example, a femoral constraint model may provide valid positions for installing an IM stem of a femoral implant within the IM canal of a femur, while maintaining implant parameters, performance metrics, placement criteria, and/or the like of the overall implant.

In one example embodiment, for a surgical planning process for a rTKA procedure, the joint (for instance, femur and/or tibia) and/or IM canal of the patient may be prepared (for instance, reamed, resected, and/or the like) by the surgeon using manual instruments. One or more tools may be used to map the (3D) volume of the IM canal to define a 3D "constraint space" for the implant stem which in turn imposes limits on implant placement. In some embodiments, if the 3D constraint space is smaller than the stem, then a geometric "best-fit" process may be used to simulate how the stem may minimally violate the canal (or, alternatively, align in a compressive-fit). The best-fit process may restrict implant placement and further limit how implants may be repositioned relative to any valid placement.

In another example, if the implant may be aligned to a valid position (for instance, a position that doesn't violate the canal space or is aligned to a least violating position) a user interface (UI or GUI) may provide controls that only allow adjustment of the implant and/or implant stem position/orientation that would maintain alignment. For example, the controls may be limited to provide rotation of the implant about its stem, provide translation along the stem axis, and/or the like.

In an example that includes an IM constraint space or canal volume that is larger than the stem, a UI can provide controls to move the implant in short increments if the resultant position would not violate the canal volume. In some embodiments, controls that would move the implant into violation can be visibly disabled or otherwise modified to limit control functions to avoid moving the implant into a violating position. In various embodiments, a UI may be provided that uses mouse input (or other pointing tool input) to "drag" the implant in an interface view that portrays the implant graphically relative to the defined canal volume and other anatomic features. For instance, movement of the mouse would move the implant in a corresponding manner, unless the boundary of the canal volume is intersected or the movement causes another violation (for example, changing a parameter to be out-of-range), in which case the movement of the implant would cease in that direction (and/or an alert would be generated).

Because rTKA implants can support a modular stem offset configuration, a planning UI according to some embodiments may include parameters that allow for assessment of implant position and fit while exploring a range of offset parameters. For example, a "clockface" description is often used to characterize the radial angle of offset direction. These angles may be shown on the implant/instrumentation, and a clockface dial may be provided for the user to manipulate with a mouse, touchscreen, and/or other input device in order to easily choose the setting for which the implant is virtually configured.

In exemplary embodiments, the UI may provide for selection or manipulation of the radial offset angle and offset distance and stem varus/valgus or flexion/extension, and when parameters are selected the implants are shown on the user interface relative to the canal volume and the anatomic landmarks represented in the corresponding anatomical model. Calculations of joint gaps may be presented while the surgeon manipulates the implant position (constrained by the canal volume) or adjusts the modular parameters. In various embodiments, joint performance parameters may be recalculated and displayed for each change of implant position, parameters, and/or the like.

In some embodiments, surgical planning processes may include an optimization process to determine an implant position and modular parameterization that maximally achieves the joint performance goals of the surgeon. An illustrative and non-limiting example of an optimization process may include a configuration space search which includes a heuristic cost function. For example, an implant position/joint offset parameterization could be selected that minimizes the calculated differences between flexion and extension gaps in the medial condyle, while maximizing the stem length width and length that fits in the canal volume, and further achieves a maximal centralization of the femoral implant between the femoral epicondyles.

In various embodiments, the optimization process may be tied to a UI control feature, for example, optimization could evaluate a current position of the implant, and the optimization process may include heuristics or other processes that minimize the deviation from the current position. For example, if a placement of the implant violates the canal, a UI control or other function may trigger a best-fit (or other optimization) process that may move the implant to a closest position to the current location that doesn't violate the canal. In one illustrative and non-limiting example, a best-fit optimization process may be implemented via a breadth-first search through a degree-of-freedom (DOF) (for example, a 6-DOF) space within a pre-set distance (for instance, translation and/or rotation).

Joint performance determined by multiple implant configurations may have similar outcomes (for instance, performance goals or metrics). Accordingly, in some embodiments, the UI may display multiple options that can be reviewed and selected by the surgeon as part of a surgical plan.

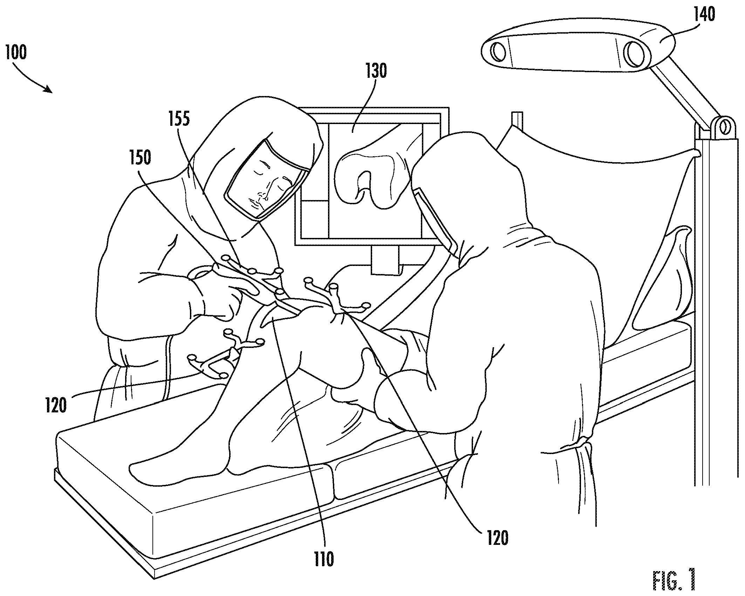

**FIG. 1** is an illustration of a system 100 for performing a revision surgical procedure using a robotic system. In some embodiments, system 100 may be or may include an image-free (for instance, CT-less) system. In other embodiments, system 100 may be or may include an image-based system based on diagnostic image data. In various embodiments, system 100 may operate using a combination of image-free and image-based processes. Embodiments are not limited in this context.

System 100 may include a surgical cutting tool 150 with an associated optical tracking frame 155 (also referred to as tracking array 155), graphical user interface 130, an optical tracking system 140, and patient tracking frames 120 (also referred to as tracking arrays 120). The illustration also includes an incision 110 through which, for example, a knee revision surgery may be performed. In an example, the illustrated robotic surgical system 100 depicts a hand-held computer-controlled surgical robotic system, for instance, the same or similar to the Navio^{®} Surgical System from Blue Belt Technologies of Plymouth, Minnesota. System 100 may use an optical tracking system 140, or other type of tracking system, coupled to a robotic controller to track and control a hand-held surgical instrument. For example, optical tracking system 140 tracks tracking array 155 coupled to surgical tool 150 and tracking arrays 120 coupled to the patient to track locations of the instrument relative to the target bone (e.g., femur and/or tibia for knee procedures).

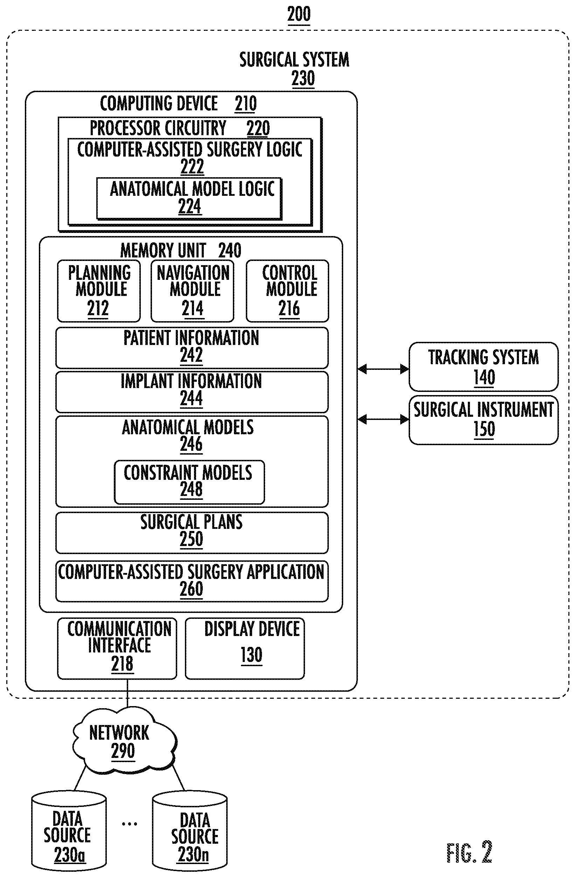

**FIG. 2** is a block diagram depicting an operating environment 200 for planning and/or performing a surgical procedure according to an example embodiment. A non-limiting example of a surgical procedure may include an orthopedic or arthroplasty procedure, for instance, a TKA or rTKA procedure.

As shown in FIG. 2, operating environment 200 may include a surgical system 230. In various embodiments, surgical system 230 may include a computing device 210 communicatively coupled to network 290 via a communication interface (for instance, a transceiver) 218. Computing device 210 may be or may include one or more logic devices, including, without limitation, a server computer, a client computing device, a personal computer (PC), a workstation, a laptop, a notebook computer, a smart phone, a tablet computing device, and/or the like. Although a single computing device 210 is depicted in FIG. 2, embodiments are not so limited, as surgical system 230 may include and/or operably communicate with, and surgery planning processes according to some embodiments may be performed using, a plurality of computing devices 210. In addition, components of computing device 210 depicted in FIG. 2 may be arranged within a plurality of different computing devices. Embodiments are not limited in this context.

In some embodiments, surgical system 230 may include computing device 210 operating for example, as a control system, a tracking system 140, and/or a surgical instrument 150. Optionally, in various embodiments, surgical system 230 may also include or may be operatively coupled to a display device 130 and/or a one or more data sources (for instance, databases) 230a-n. In some embodiments, display device 130 and/or databases 230a-n may be used to provide navigation and control of the surgical instrument 150, which may include navigation and control of a cutting tool, a point probe, or other tools/instruments, that may be used during an arthroplasty procedure, such as an orthopedic (or similar) prosthetic implant revision surgery.

Control system 210 may include one or more computing devices configured to coordinate information received from tracking system 130 and provide control to surgical instrument 150. In some embodiments, control system 210 may include a planning module 212, a navigation module 214, a control module 216, and a communication interface 218. In certain embodiments, planning module 212 may provide pre-operative planning services that allow surgeons or other healthcare professionals to virtually plan a procedure prior to reshaping the target joint during the revision procedure on the patient.

In an example, such as for an rTKA procedure, planning module 212 may be used to manipulate a virtual model of the implant (for instance, an "implant model") in reference to a virtual implant host model (for instance, an "anatomical model"). In some embodiments, the virtual model of the implant host (for instance, the joint to be revised, such as a knee joint and/or components thereof) may be created through use of a point probe or similar instrument tracked by the optical tracking system 140. Control system 210, for instance, via planning module 212, may be used to collect data from surfaces of the target joint to create an anatomical model in the form of a virtual model of the patient's actual anatomical structure. Particularly in a revision surgery, this method may, among other things, increase the accuracy of the planning process by using data collected after the existing implants are removed and without intra-operative imaging. Collecting surface data from the target bone(s) may also allow for iterative reshaping of the target bone to ensure proper fit of new prosthetic implants and optimization of anatomical alignment.

In an example, navigation module 214 may coordinate tracking the location and orientation of the implant, the implant host, and/or surgical instrument 150. In certain examples, navigation module 214 may also coordinate tracking of the virtual models used during pre-operative or intra-operative planning within planning module 212. Tracking the virtual models may include operations such as alignment of the virtual models with the implant host through data obtained via the tracking system 140. In these examples, navigation module 214 receives input from tracking system 140 regarding the physical location and orientation of surgical instrument 150 and an implant host. Tracking of the implant host may include tracking multiple individual bone structures, such as with tracking arrays 120. For example, during a TKA procedure, tracking system 140 may individually track the femur and the tibia using tracking devices anchored to the individual bones (for example, as illustrated in FIG. 1).

In an example, control module 216 may process information provided by navigation module 214 to generate control signals for controlling surgical instrument 150. In certain examples, control module 216 may also work with navigation module 214 to produce visual animations to assist the surgeon during an operative procedure. Visual animations may be displayed via a display device, such as display device 130. In an example, the visual animations may include real-time or substantially real-time 3D representations of the implant, the implant host, and/or surgical instrument 150, among other things. In certain examples, the visual animations may be color-coded to further assist the surgeon with positioning and orientation of the implant.

In various embodiments, communication interface 218 may facilitate communication between control system 210 and external systems and devices. Communication interface 218 may include both wired and wireless communication interfaces, such as Ethernet, IEEE 802.11 wireless, or Bluetooth, among others. As illustrated in FIG. 1, in this example, the primary external systems connected via the communication interface 218 may include tracking system 140 and surgical instrument 150. Although not shown, databases 230a-n and display device 130, among other devices, may also be connected to control system 210 via communication interface 218. In an example, communication interface 218 may communicate over an internal bus to other modules and hardware systems within control system 210.

In exemplary embodiments, tracking system 140 may provide location and orientation information for surgical devices and parts of an implant host's anatomy to assist in navigation and control of semi-active robotic surgical devices. Tracking system 140 may include a tracker (e.g., tracking array 120) that includes or otherwise provides tracking data based on at least three positions and at least three angles. The tracker may include one or more first tracking markers associated with the implant host, and one or more second markers associated with the surgical device (e.g., surgical instrument 150). The markers or some of the markers may be one or more of infrared sources, Radio Frequency (RF) sources, ultrasound sources, transmitters, and/or the like. Tracking system 140 may be or may include an infrared tracking system, an optical tracking system, an ultrasound tracking system, an inertial tracking system, a wired system, and/or a RF tracking system. One illustrative and non-limiting example tracking system may be the OPTOTRAK^{®} 3-D motion and position measurement and tracking system described herein, although those of ordinary skill in the art will recognize that other tracking systems of other accuracies and/or resolutions may be used.

In an example, a surgeon may use surgical instrument 150 to shape a target bone to accommodate a new prosthetic implant in place of an existing implant that failed. For example, as discussed above, it is not uncommon to need to revise a TKA by removing the prosthetic implants forming the artificial knee, reshape the femur and tibia, and implant new prosthetic implants to form a new artificial knee. In order to assist in performing a revision, control surgical system 230 may track and control a surgical cutting instrument to perform precise cuts according to a virtual plan generated after removal of the old prosthetic.

In some embodiments, control system 210 may operate to generate and/or access virtual or anatomical models of a patient. For example, control system 210 may operate to generate anatomical models of a patient via an anatomical model generation process the same or similar to the methods described in U.S. Patent Application Publication No. 2019/0365474, titled "Systems and Methods for Planning and Performing Image Free Implant Revision Surgery," and/or PCT International Application No. PCT/US2020/054231, titled "Registration of Intramedullary Canal During Revision Total Knee Arthroplasty," both of which are incorporated by reference in the present disclosure as if fully written herein. Embodiments are not limited in this context. For example, surgical planning processes according to some embodiments may generate and/or access anatomical models created via various other processes (for instance, image-based processes) capable of operating with embodiments described in the present disclosure.

The anatomical models may be or may include virtual representations of portions of the patient, such as the knee joint (including, for instance, the joint portions of the distal femur and/or proximal femur that interface as part of the knee joint), for example, the same or similar to the computer models of patient anatomy provided in the Navio^{®} Surgical System. The anatomical models may be graphically depicted via display device 130 and visually manipulated (for instance, rotated, moved, viewed wholly or partially transparent or semitransparent, viewed as wireframe or similar images, and/or the like).

In one non-limiting example, the anatomical model generation process may include an image-free process using a point probe (for instance, an optically tracked point probe) to map the actual surface of the target bone(s) that need a new implant. Mapping may be performed after removal of the defective or worn-out implant, as well as after removal of any diseased or otherwise unwanted bone. Points may be collected on the bone surfaces via "painting" by brushing or scraping the entirety of the remaining bone with the tip of the point probe. The collected points may be used to create a three-dimensional model or surface map of the bone surfaces in the computerized planning system. In another non-limiting example, an anatomical model may be mathematically accomplished by capturing a series of Cartesian coordinates that represent the tissue surface, for instance, to generate a model file (for instance, without limitation, *.stl, *.obj., *.fea, *.stp, *.sur, *.igs, *.wrl, *.xyz, and/or the like file formats). In an additional non-limiting example, the anatomical model generation process may include an image-based process based on diagnostic images of the subject anatomy of a patient, such as X-ray images, CT images, and/or the like. Image analysis software may be used to analyze the diagnostic images to generate anatomical models, such as 3D models. In some embodiments, the anatomical model generation process may use a combination of image-free and image-based processes. In general, anatomical models according to some embodiments may be generated using various processes, including, without limitation, traditional probe painting, 3D imaging mapped with references, visual edge detection, combinations thereof, and/or the like.

For example, pre- or intra-operatively, control system 210 may create a two-dimensional (2D) or 3D anatomical model of one or more portions of the patient, including a femur and/or tibia for a TKA or rTKA procedure. The anatomical model may be generated based on information specific to the patient, such as anatomical dimensions of the bony anatomy of interest of the patient, the mechanical and anatomical axes of the leg bones, ends of the distal femur and proximal tibia, the epicondylar axis, the femoral neck axis, the dimensions (e.g., length) of the femur and/or tibia, the location of anatomical landmarks such as the lesser trochanter landmarks, the distal landmark, combinations thereof and/or the like.

As discussed herein, during certain surgeries, in particular rTKA procedures, reference may be made to the IM canal of the femur and/or the tibia when placing one or more implant components. Moreover, conventional implant systems may utilize the IM canal as a primary mode of fixation and/or attachment once the component is implanted (e.g., by attaching a stem to the femoral or tibial implant). When implants containing stems are used, the stems may often constrain the position of the implant relative to the native anatomy in several degrees of freedom, namely flexion/extension rotation, varus/valgus rotation, and potentially medial/lateral (M/L), anterior/posterior (A/P) position, and/or the like. Accordingly, in some embodiments, anatomical models may be or may include IM canal information. For example, tracking array 140 (for instance, via a point probe) may be used as part of an IM canal registration process to collect information related to the IM canal for generating a corresponding anatomical model and/or portions thereof.

For a particular surgical procedure, such as an rTKA procedure, the procedure or episode of care can include three phases: pre-operative, intra-operative, and post-operative. During each phase, data may be collected or generated that can be used to analyze the episode of care in order to understand various features of the procedure and identify patterns that may be used, for example, in training models to make decisions with minimal human intervention. The data collected over the episode of care may be stored at the control system 210 and/or databases 230a-n as a complete dataset. Thus, for each episode of care, a dataset exists that may include some or all of the data collectively pre-operatively about the patient, all of the data collected or stored by control system intra-operatively, and any post-operative data provided by the patient or by a healthcare professional monitoring the patient.

During the registration process, for example, display device 130 can show a preoperatively constructed 3D bone anatomical model and depict the locations of the probe as the surgeon uses the probe to collect locations of anatomical landmarks on the patient. In some embodiments, the Display device 130 can include information about the surgical target area. For example, in connection with a TKA, display device 130 can depict the mechanical and anatomical axes and/or IM canal of the femur and tibia. Display device 130 may depict varus and valgus angles for the knee joint based on a surgical plan, and display device 130 can depict how such angles will be affected if contemplated revisions to the surgical plan are made. Accordingly, display device 130 may operate as an interactive interface that can dynamically update and display how changes to the surgical plan would impact the procedure and the final position and orientation of implants installed on the patient anatomy.

Modules directed to ligament and gap balancing, for example, may include pre-and post-resection ligament/gap balancing, and the surgeon may select which modules to include in their default surgical plan workflow depending on whether they perform such ligament and gap balancing before or after (or both) bone resections are performed.

Computing device (or control system) 210 may include a processor circuitry 220 that may include and/or may access various logics for performing processes according to some embodiments, for example, a surgical planning process. For instance, processor circuitry 220 may include and/or may access a computer-assisted surgery logic 222 and/or an anatomical model logic 224. Processing circuitry 220, computer-assisted surgery logic 222, anatomical model logic 224, and/or portions thereof may be implemented in hardware, software, or a combination thereof. As used in this application, the terms "logic," "component," "layer," "system," "circuitry," "decoder," "encoder," "control loop," and/or "module" are intended to refer to a computer-related entity, either hardware, a combination of hardware and software, software, or software in execution, examples of which are provided by the exemplary computing architecture 900. For example, a logic, circuitry, or a module may be and/or may include, but are not limited to, a process running on a processor, a processor, a hard disk drive, multiple storage drives (of optical and/or magnetic storage medium), an object, an executable, a thread of execution, a program, a computer, hardware circuitry, integrated circuits, application specific integrated circuits (ASIC), programmable logic devices (PLD), digital signal processors (DSP), field programmable gate array (FPGA), a system-on-a-chip (SoC), memory units, logic gates, registers, semiconductor device, chips, microchips, chip sets, software components, programs, applications, firmware, software modules, computer code, a control loop, a computational model or application, an AI model or application, an ML model or application, a proportional-integral-derivative (PID) controller, FG circuitry, variations thereof, combinations of any of the foregoing, and/or the like.

Although computer-assisted surgery logic 222 is depicted as being within processor circuitry 220 and anatomical model logic 224 is depicted as being within computer-assisted surgery logic 222 in FIG. 1, embodiments are not so limited. For example, computer-assisted surgery logic 222, anatomical model logic 224, and/or any component thereof may be located within an accelerator, a processor core, an interface, an individual processor die, a memory, a storage device, a data store, a database, implemented entirely or partially as a software application (for instance, a computer-assisted surgery application 260), and/or the like.

Memory unit 240 may include various types of computer-readable storage media and/or systems in the form of one or more higher speed memory units, such as read-only memory (ROM), random-access memory (RAM), dynamic RAM (DRAM), Double-Data-Rate DRAM (DDRAM), synchronous DRAM (SDRAM), static RAM (SRAM), programmable ROM (PROM), erasable programmable ROM (EPROM), electrically erasable programmable ROM (EEPROM), flash memory, polymer memory such as ferroelectric polymer memory, ovonic memory, phase change or ferroelectric memory, silicon-oxide-nitride-oxide-silicon (SONOS) memory, magnetic or optical cards, an array of devices such as Redundant Array of Independent Disks (RAID) drives, solid state memory devices (e.g., USB memory, solid state drives (SSD) and any other type of storage media suitable for storing information. In addition, memory unit 240 may include various types of computer-readable storage media in the form of one or more lower speed memory units, including an internal (or external) hard disk drive (HDD), a magnetic floppy disk drive (FDD), and an optical disk drive to read from or write to a removable optical disk (e.g., a CD-ROM or DVD), a solid state drive (SSD), and/or the like.

Memory unit 240 may store various types of information and/or applications for performing features and processes according to some embodiments, for instance, a surgical planning process according to some embodiments. For example, memory unit 240 may store patient information 242, implant information 244, anatomical models 246, constraint models 248, surgical plans 250, and/or computer-assisted surgery application 260. In some embodiments, all or some of the information depicted as being stored in memory unit 240 may be, in whole or in part, stored in data sources 230a-n and accessible to computing device 210.

In some embodiments, patient information 242 may include information for a patient undergoing a surgical procedure being performed via surgical system. Patient information 242 may include personal information (for instance, name, address, and/or the like), physical characteristics (for instance, height, weight, and/or the like), medical information (for instance, health history, health record identifiers, procedure information, and/or the like), and/or any other type of information that may be associated with a patient. In various embodiments, patient information 242 may include information associated with a population of patients. For example, the population of patients may include individuals that may be used as a comparison with a patient undergoing a procedure in order to generate anatomical models, constrain models, surgical plans, and/or the like according to some embodiments. Patient information 242 for the population of patients may include any type of information that may be used by surgical system 230 to perform processes according to some embodiments. For example, patient information 242 may include outcomes and/or performance information for individuals that have had a rTKA procedure with certain parameters (for instance, varus/valgus angles, implant fit deviations, and/or the like).

In various embodiments, implant information 244 may include information associated with an implant associated with a surgical procedure being performed via surgical system 230. In general, implant information 244 may include information required for modeling an implant and/or determining a configuration of an implant on or within patient anatomy. For example, implant information 244 may include information related to a femur component of a knee implant for a rTKA procedure, including, without limitation, dimensions, recommended parameters, stem information, manufacturer instructions, fit tolerances, and/or the like.

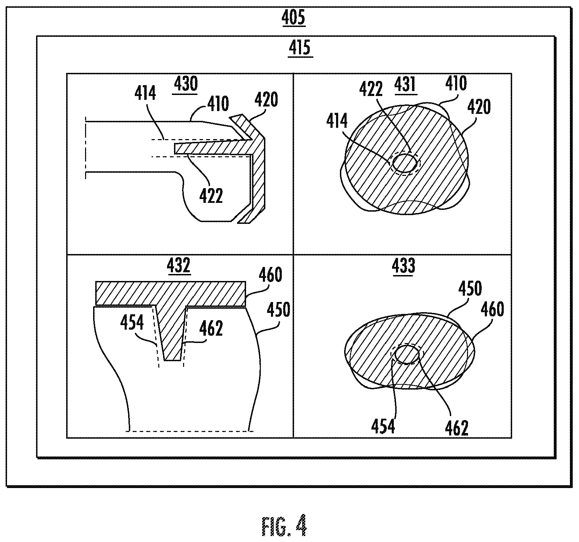

In exemplary embodiments, anatomical models 246 may include virtual models of patient anatomy associated with a surgical procedure being performed via surgical system 230. In some embodiments, the virtual models may include mathematical models, graphical models, feature maps, combinations thereof, and/or the like configured to virtually represent patient anatomy within a computing system (see, for example, FIG. 4).

In some embodiments, anatomical models 246 may include constraint models 248. In various embodiments, a constraint model 248 may include constraint space that includes a virtual representation of valid placement information for an implant within an anatomical model. For example, an anatomical model 246 may include a portion of a femur configured to receive a femur component of an implant. A corresponding constraint model 248 may be generated to represent, indicate, determine, or otherwise provide valid (or alternatively, invalid) placement locations, options, parameters, and/or the like. For example, for an rTKA procedure, the IM canal of the patient may be prepared by the surgeon using manual instruments. A computer-assisted tool (for instance, tracking system 140) may be used to map the femur and the 3D volume of the femoral IM canal to generate an anatomical model 246 of the femur. A constraint model 248 may be generated that defines a 3D constraint space for a femoral implant stem for placement within the femoral IM canal, which in turn imposes limits on implant placement.

In some embodiments, constraint spaces 248 may be generated based only on the physical anatomy of the corresponding patient anatomy (for instance, the dimensions of the IM canal of a patient). In other embodiments, constraint spaces 248 may be generated based on the corresponding patient anatomy and implant information (for instance, dimensions, parameters, and/or the like). In various embodiments, constraint spaces 248 may be generated based on the corresponding patient anatomy, implant information, and implant conditions (for instance, parameter requirements (for example, a varus/valgus angle between X degrees and Y degrees), performance requirements, press- or friction-fit requirements or tolerances, and/or the like).

In some embodiments, a surgical plan 250 may include instructions, methods, steps, workflows, and/or the like for performing a surgical procedure. In various embodiments, surgical system 230 may develop a surgical plan 250 based on anatomical models 246 and/or constraint models 248 (for instance, 3D models of components of the knee joint) and other information specific to the patient (for instance, patient information 242), such as the mechanical and anatomical axes of the leg bones, dimensions of the femur and tibia, the location of anatomical landmarks such as the lesser trochanter landmarks, and/or the like. Surgical plans 250 may provide a recommended optimal implant size, implant position, implant orientation, implant parameters, and/or the like based on anatomical models 246, constraint models 248, and/or patient information 242. Surgical plans 250 can include proposed details on offset values, varus/valgus angles, implant parameters, gap planning, femoral stem sizing and length, tibial stem sizing and length, and/or the like.

Surgical plans 250 may be viewed preoperatively and intraoperatively, and the surgeon can modify surgical plans 250 preoperatively or intraoperatively. Surgical plans 250, for example, can display the planned resection of a femur and/or tibia and superimpose the planned implants onto corresponding anatomical models 246 and/or constraint models 248 on the planned resections. Surgical system 230 can provide the surgeon with options for different surgical workflows that will be displayed to the surgeon based on a surgeon's preference. For example, the surgeon can choose from different workflows based on the number and types of anatomical landmarks that are checked and captured and/or the location and number of tracker arrays used in the registration process. Embodiments are not limited in this context.

Surgical plans 250 may be administered, in whole or in part, manually by a surgeon, automatically (for instance, computer- or robot-assisted) via computer-controlled surgical instruments 150, and/or combinations thereof. Embodiments are not limited in this context.

In some embodiments, computer-assisted surgery logic 222 may operate to perform, implement, or otherwise provide computer-assisted surgery processes for surgical system 230 according to some embodiments. For example, in various embodiments, computer-assisted surgery logic 222 may operate to manage or control operational features of planning module 212, navigation module 214, control module 216, tracking system 140, and/or surgical instruments 150 described in the present disclosure. In another example, computer-assisted surgery logic 222 may perform, implement, or otherwise provide surgical planning processes according to some embodiments.

In exemplary embodiments, anatomical model logic 224 may operate to perform, implement, or otherwise provide anatomical models 246 and/or constraint models 248 according to various embodiments. For example, anatomical model logic 224 may receive patient information 242, implant information 244, and/or information from tracking system 140 to generate anatomical models 246 and/or constraint models as described in the present disclosure.

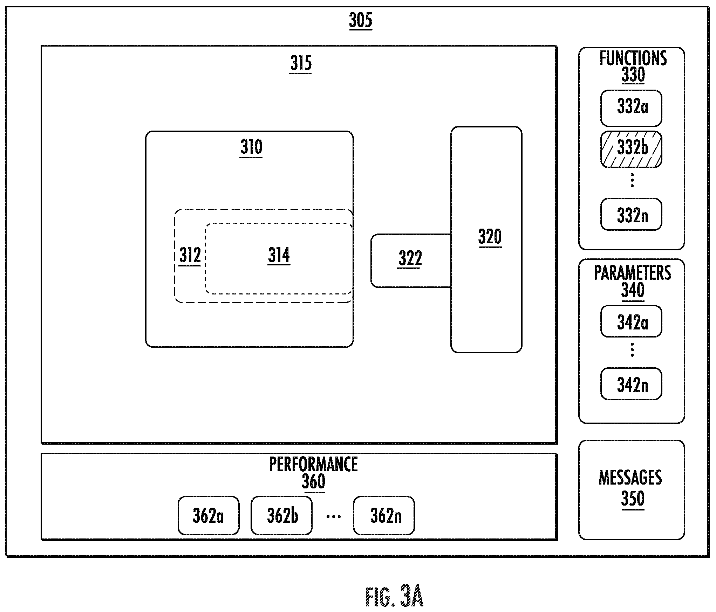

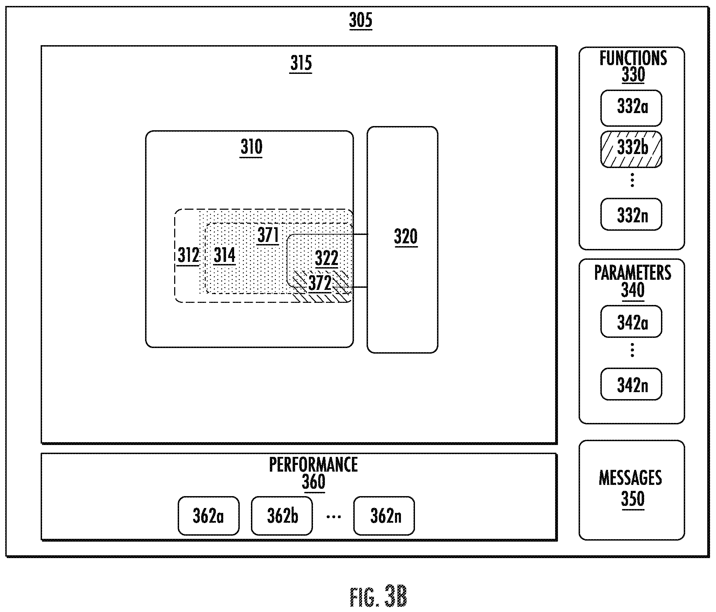

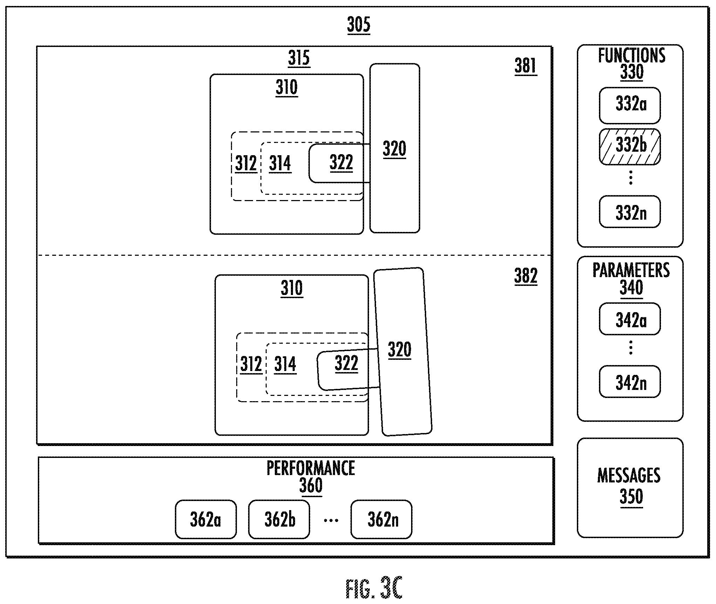

In some embodiments, computer-assisted surgery application 260 may be or may include a software application that includes and/or operates in combination with computer-assisted surgery logic 222 and/or anatomical model logic 224 to perform features of surgical system 230 described in the present disclosure. For example, computer-assisted surgery application 260 may operate to present user interfaces (see, for example, FIGS. 3, 4, and 6) via display device 130.

In some embodiments, some or all of patient information 242, implant information 244, anatomical models 246, constraint models 248, surgical plans 250, computer-assisted surgery logic 222, anatomical model logic 224, and/or a computer-assisted surgery application 260 may be stored in one or more data sources 230a-n accessible to computing device 210 via network 290.

**FIG. 3A** depicts an illustrative graphical user interface for performing a surgery planning process in accordance with one or more features of the present disclosure. As shown in FIG. 3A, a UI screen 305 may be presented to a user, such as a surgeon, as part of a surgical planning process according to various embodiments. UI screen 305 may be presented via computer-assisted surgery logic 222, anatomical model logic 224, and/or a computer-assisted surgery application 260.

In various embodiments, UI screen 305 may include a model UI 315 operative to display an anatomical model 310 and an implant 320 generated according to some embodiments. For example, UI screen 305 may be for an rTKA procedure and anatomical model 310 may be a portion of the distal femur or proximal tibia, and implant 320 may be a femoral component or tibial component, respectively, of a knee implant device. In some embodiments, anatomical model 310 may include a model interface 312 configured to interface with a corresponding implant interface 322 of an implant 320. In general, model interface 312 may be any portion of a represented anatomy that engages an installed implant and, therefore, is part of the evaluation of the fit between the implant and the bony anatomy. For example, model interface 312 may be an IM canal, a resected surface of a femur or tibia, femoral condyles, fibular head, tibial tubercle, and/or any other portion of bony anatomy that may affect the fit of an implant. For instance, in FIG. 3, model interface 312 may be in the form of an IM canal arranged within a femur (or tibia) model 310 configured to engage a stem 322 of a femoral (or tibial) implant component 320. Although model interface 312 is depicted as being arranged within model 310, embodiments are not so limited, as model interface may be external to model (for instance, a resected outer surface, an epicondyle, and/or the like).

In one example of an rTKA procedure, an IM canal of a femur may be prepared by a surgeon (manually, with computer- or robot-assistance, or a combination thereof) and an anatomical model of the femur generated according to some embodiments (for instance, via probe painting, 3D imaging mapped with references, visual edge detection, combinations thereof, and/or the like). As part of generating the anatomical model, the IM canal may be registered according to various embodiments to generate model 310 with model interface 315 of the IM canal.

In various embodiments, a constraint model (or constraint space) 314 may be generated for the model interface 312. In general, constraint model 314 may define the valid placement configurations, locations, areas, regions, zones, or other arrangements of implant 320 in association with model 310. A valid placement configuration may include a position of implant 320 that comply with one or more implant placement conditions, including, without limitation, fitting on or within anatomy (for instance, an implant stem of a tibial component fitting within the physical dimensions of the IM canal of the tibia), performance criteria or goals (for instance, placement of the implant may be required to meet certain performance metrics, such as range of motion, gait, deformities, and/or the like), placement criteria or goals (for instance, gap placement criteria, varus/valgus angle criteria, and/or the like), combinations thereof, and/or the like. In some embodiments, model 310 may be associated with a plurality of constraint models 314 (for instance, a constraint space for an IM canal, a constraint space for a resected surface, and/or the like).

In some embodiments, therefore, constraint model 314 may define the possible placement configurations that meet the implant placement conditions. For example, constraint model 314 may define one or more configurations where implant interface 322 may physically fit within model interface 312 while also meeting user-specified conditions, such as varus/valgus angle, gap calculations, performance metrics, and/or the like. Accordingly, constraint model 314 may operate to remove invalid implant configurations so that a user does not waste time or resources evaluating implant configurations that will not be used in an actual surgical procedure.

In various embodiments, anatomical model 310, model interface 312, and/or constraint model 314 may not model or represent the entirety of the corresponding anatomical portion of the patient. For example, anatomical model 310, model interface 312, and/or constraint model 314 may only represent or visualize a portion of an anatomical portion required for evaluating implant fit or other considerations of a surgical procedure.

In some embodiments, UI screen 305 may include a function object 330 for providing various functions 332a-n for interacting with model 310 and/or implant 320. For example, functions 332a-n may include objects for allowing a user to manipulate model 310 and/or implant 320, such as movement in space (for instance, 3D movement), rotation, panning, zooming, setting view type (for instance, transparency, wireframe images, and/or the like), removing/adding visual layers, and/or the like. In some embodiments, functions 332a-n may be associated with GUI objects, such as a button for moving model 320. In various embodiments, functions 332a-n may not be directly associated with GUI objects, such as using a mouse, touch screen, or other input device to allow a user to select (grab or hold) and move implant 320

In some embodiments, UI screen 305 may include a function object 330 for providing various functions 332a-n for interacting with model 310 and/or implant 320. For example, functions 332a-n may include objects for allowing a user to manipulate model 310 and/or implant 320, such as movement in space (for instance, 3D movement), rotation, panning, zooming, setting view type (for instance, transparency, wireframe images, and/or the like), removing/adding visual layers, and/or the like. In some embodiments, functions 332a-n may be associated with GUI objects, such as a button for moving model 320. In various embodiments, functions 332a-n may not be directly associated with GUI objects, such as using a mouse, touch screen, or other input device to allow a user to select (grab or hold) and move implant 320.

In various embodiments, UI screen 305 may include a parameters object 340 to allow users to enter parameters 342a-n for implant 320, including, without limitation varus/valgus angle, flexion/extension angles, offsets, performance goals, gap calculations, placement violation thresholds, and/or the like. In some embodiments, changes to parameters 342a-n may cause corresponding changes to constraint model 314. For example, changing a varus/valgus angle for implant 320 may change the valid placement configurations for implant 320 and, therefore, constraint model 314.

In some embodiments, functions 332a-n may be locked, limited, or otherwise made unavailable if activation would violate constraint model 314. For example, if selection of an up/down movement button would cause implant 320 to violate a side wall of model interface 312 (for instance, cross over a surface of an IM canal), then the up/down movement button may be disabled. In some embodiments, parameters 332a-n may be locked, limited, or otherwise made unavailable if a parameter value would violate constraint model 314. For example, if an offset angle is required to be at a specific value, then parameter 342a-n may not allow user input. In another example, if an offset angle is required to be within a range of X to Y, then a user may be limited to only setting the offset angle to a value between X and Y. In various embodiments, a user may be allowed to override any limitations.

In various embodiments, UI screen 305 may include a messages object 350 for presenting messages and other information to a user. In some embodiments, messages 350 may include alerts, alarms, or other information associated with placement of implant 320 within anatomical model 310. For example, if a user moves implant 320 into a position and/or inputs a parameter that violates constraint model 314, an alert may be presented via messages object 350.

Some embodiments may provide additional messaging features. For example, referring to **FIG. 3B**, one or more validity indicators 371, 372 may be presented in association with model 310, implant 320, and/or portions thereof. For example, indicator 371 may be overlayed on model 310, such as over constraint model 314. A color or other visual feature of indicator 371 may be used to indicate whether the placement or other configuration of implant 320 violates constraint model 314. For instance, if the configuration of model 320 does not violate constraint model 314, then indicator 371 may be green (or have another visual characteristic to visually indicate that model 320 is in compliance with the conditions associated with constraint model 314, such as an overlayed pattern, a highlighted boundary, flashing graphics, and/or the like). If the configuration of model violates a condition, then indicator 371 may turn red (or have another visual characteristic to visually indicate that model 320 is not in compliance with the conditions associated with constraint model 314).

In another example, indicator 372 may indicate a specific area of violation. For instance, if implant interface 322 is moved to violate constraint model 314, indicator 372 may be activated to point out a specific area of the violation (for instance, contacting a specific portion of an inner wall of an IM canal). Indicators 371 and 372 may be used in combination with other information such as messages 350. For example, indicator 371 and/or 372 may change color, and messages 350 may present information detailing a violation.

Although indicators 371 and 372 are depicted in FIG. 3B as being graphical objects overlaying portions of model 310 and/or implant 320, embodiments are not so limited. For example, indicator 371 and/or 372 may be arranged in various locations within UI screen 305. In other embodiments, an indicator may include other forms of communicating compliance/non-compliance with placement conditions, such as an auditory, light, tactile, or other indicator.

Accordingly, in some embodiments, movement of model 320 and/or parameters that may affect model 320 (for instance, radial offset parameters) may be bound within the confines defined by constraint model 314 (for instance, "non-free" movement). In various embodiments, in a non-free movement embodiments, violating movements may be prevented and/or the model may be moved in a snap-back manner to a previous, non-violating position once the input (for instance, mouse selection) is removed. In some embodiments, indicators (for instance, indicators 371 and 372), alerts, messages, and other information may be used in combination with non-free movement of model 320 (for instance, movement of model 320 is limited to constraint space 314). In other embodiments, free movement (for instance, movement of model 320 is not bound by constraint space) may be allowed in combination with indicators, alerts, and/or the like. Accordingly, embodiments may operate in a free and/or a non-free movement model, for example, that may be selectable by a user.

In some embodiments, UI screen 305 may include performance objects 360 configured to indicate performance information or metrics associated with a configuration of implant 320. Non-limiting examples of performance information 362a-n may include gap calculations, range of motion, gait, deformities, and/or the like. In various embodiments, performance information 362a-n may be updated responsive to a user changing the configuration of model 320, including changing parameters 342a-n. For example, if a user moves implant 320 axially within anatomical model 310, a gap calculation performance information 362a-n may be recalculated and displayed to indicate how the change in the configuration of implant 320 may affect implant performance.

In some embodiments, functions 332a-n may include one or more optimization functions. Referring to FIG. 3C, a user may select an optimization function 332a-n, and one or more optimized configuration 381, 382 may be presented via model UI 315. In general, optimized configurations 381, 382 may include a configuration of model 320 that does not violate constraint space 314. Optimization may be based on one or more specific criteria, such as a performance metric, an offset angle, and/or the like that may be specified by a user and/or a particular surgical workflow.

In some embodiments, for example, an optimization process may be or may include a configuration space search which includes a heuristic cost function. In various embodiments, an optimization process may be used to determine an implant position and modular parameterization that maximally achieves the joint performance goals of the surgeon. For example, an optimization process may operate to maximize the stem width that fits within an IM canal volume. In another example, an optimization process may operate with implant position/joint offset parameterization selected that minimizes the calculated differences between flexion and extension gaps in the medial condyle, while maximizing the stem length width and length that fits in the canal volume, and further achieves a maximal centralization of the femoral implant between the femoral epicondyles.

In various embodiments, optimization may be or may include a "best-fit" process. For example, if an IM stem of an implant is larger or otherwise conflicts with the available space within the IM canal modeled via model 310, then a best-fit process may be used to simulate how the stem may minimally violate the canal. In some embodiments, multiple violations may be managed through the best-fit process, for example, through weighting and/or the like (for instance, higher-weighted violations may be addressed before and/or to a greater extent than lower-weighted violations). In one non-limiting example, a best-fit process may be or may include aligning the stem in a compressive-fit configuration. In another non-limiting example, a best-fit process (for a press-fit IM stem, for instance) may include optimizing based on a degree-of-freedom of the IM stem (for instance, restricting the degree-of-freedom) (e.g., forcing the IM stem and IM canal axes to be coaxial (or coaxial within a threshold)). Accordingly, for example, configurations 381 and 382 may include best-fit configurations.

In some embodiments, an optimization process, including a best-fit process, may operate based on a current position of implant 320 with respect to anatomical model 310. For example, an optimization process may determine an optimal configuration while minimizing movement of implant 320 and/or changes to parameters 342a-n from current values. In another example, an optimization process may take into account a current position of implant 320 and use heuristics that minimize the deviation from the current position.

**FIG. 4** depicts an illustrative graphical user interface for performing a surgery planning process in accordance with one or more features of the present disclosure. As shown in FIG. 4, a UI screen 405 may include a model UI 415 for presenting various anatomical models in one or more views. For example, model UI 415 may include a side view 430 and a top-down view 431 of a femur model 410 having a constraint model 414 associated with an IM canal of the modeled femur of a patient. Femur model 410 may be associated with a femoral component model 420 having a stem 422 configured to be arranged within an IM canal associated with constraint space 414. Model UI 415 may include a side view 432 and a top-down view 432 of a tibia model 450 having a constraint model 454 associated with an IM canal of the modeled tibia of a patient. Tibia model 450 may be associated with a tibial component model 460 having a stem 462 configured to be arranged within an IM canal associated with constraint space 454.

Implants 420 and 460 may include various implants now known and/or developed in the future. Referring to **FIGS. 5A** and **5B**, therein is depicted illustrative and non-limiting examples of implants that may be used according to some embodiments. In particular, FIG. 5A is an exploded, perspective view of an example of an embodiment of a knee prosthesis (e.g., a femoral component or implant) in accordance with one feature of the present disclosure, and FIG. 5B is an exploded, perspective view of an example of an embodiment of a knee prosthesis (e.g., a tibial component or implant) in accordance with one feature of the present disclosure. The implants depicted in FIGS. 5A and 5B are the same or substantially similar to implants described in PCT International Application No. PCT/US2020/044273, titled "Orthopaedic Implant," which is incorporated by reference in the present disclosure as if fully written herein.

Referring to FIG. 5A, an example of an embodiment of a knee prosthesis in accordance with one or more features of the present disclosure is shown. As shown, the knee prosthesis may be in the form of a femoral implant 510. Femoral implant 510 may include a femoral load bearing component 512 (e.g., a condylar component), an intermedullary stem 514, and an intermediate stem extension or coupler 516. In one embodiment, coupler 516 may be omitted and stem 14 may be directly coupled to the femoral load bearing component 512. In other embodiments, coupler 16 may be integrally formed with stem 514 or femoral load bearing component 512. For example, stem 14 and coupler 516 may be monolithic. As illustrated, in use, coupler 516 connects femoral load bearing component 512 and stem 514.

Similarly, referring to FIG. 5B an alternate example of an embodiment of a knee prosthesis in accordance with one or more features of the present disclosure is shown. As shown, the knee prosthesis may be in the form of a tibial implant 520. Tibial implant 520 may include a tibial load bearing component 522 (e.g., a tibial tray or platform), an intermedullary stem 524, and an intermediate stem extension or coupler 516. In one embodiment, coupler 516 may be omitted and stem 524 may be directly coupled to tibial load bearing component 522. In other embodiments, coupler 16 may be integrally formed with stem 524 or tibial load bearing component 522. For example, stem 524 and coupler 516 may be monolithic. As illustrated, in use, coupler 516 connects the tibial load bearing component 522 and stem 524.

In some embodiments, constraint modeling may be configured to manage implants the same or similar to implants 510 and/or 520 and associated parameters.

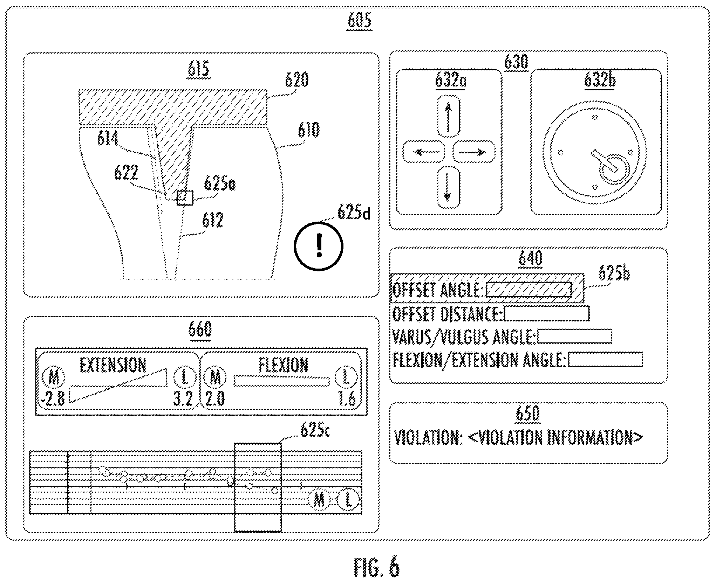

**FIG. 6** depicts an illustrative graphical user interface for performing a surgery planning process in accordance with one or more features of the present disclosure. As shown in FIG. 6, a UI screen 605 may include a model UI 615 presenting a tibial anatomical model 610. According to some embodiments, anatomical model 610 may include a registered IM canal 612 be associated with a constraint model 614.

UI screen may include functions 630, such as 632a and 632b, for moving a tibial implant component 620 with respect to anatomical model 610. For example, function 632a may be used to move implant 320 (or, alternatively, anatomical model 610) in short increments in various directions, for instance, if the resultant position would not violate constraint model 614. In another example, function 632b may include a clockface dial to allow a user to manipulate clock-based parameters of model 610, such as a radial angle of an offset direction. For instance, in reference to implants 510 and 520, clockface dial 632b may be used to easily and efficiently set the offset of coupler 16 through various clock positions. In some embodiments, as clockface dial 632b is moved, various corresponding parameters (such as varus/valgus angle, flexion/extension angle, and/or the like) may also be modified to achieve a valid set of associated parameters. By way of a non-limiting example, 4 degrees of varus/valgus angle and 4 degrees of flexion/extension angle may be selected responsive to clockface dial being moved to a position of 3 O'clock (for instance, 45 degrees). In one embodiment, for example, where an IM stem axis is fixed/constrained (e.g., in a press-fit configuration), implant 'clocking' and sizing may be optimized for implant/bone coverage and to minimizing overhang rather than, for example, optimizing for varus/valgus, flexion/extension, and/or other angles.

In some embodiments, UI screen 605 may include parameters 640, performance information 660, and/or messages 650. According to various embodiments, one or more indicators 625a-d may be presented to indicate an invalid implant configuration. For example, indicator 625a may indicate that stem 622 is violating an inner wall of constraint model 614 (and, in the depicted example, also IM canal 622). In another example, indicator 625b may indicate that the current configuration violates an offset angle parameter. In a further example, indicator 625c may indicate that a current configuration violates a projected performance metric 660, such as a flexion performance metric.

In some embodiments, one or more of indicators 625a-d may be configured as a "bone preparation indicator" configured to indicate a violation associated with preparing a bone, for example, associated with anatomical model 610. For example, 625d may be a notching indicator presented responsive to determining that the user has planned for tibial implant component 620 to be in a position, orientation, and/or the like that may result in "notching" of the IM canal 612. A surgical planning process according to various embodiments may operate to determine (roughly, within threshold tolerances, and/or the like) whether a saw cut would end inside or outside the bone shaft surface of IM canal 612 according to the bone preparation due to the positioning of tibial implant component 620. "Notching" may lead to a notched cut in the bone, which might be a stress riser, increasing, for example, the chance of a bone fracture. If a user re-positions tibial implant component such that the notching violation is not present, indicator 625d may no longer be presented on UI screen 605.

Although bone preparation indicators, such as indicator 625d, are described in the present disclosure in relation to an example associated with tibial implants, "notching," and saw preparation cuts/tools, embodiments are not so limited. For example, bone preparation indicators may be used in relation to other bone structures (for instance, the femur), implants, preparation violations, and/or preparation tools (e.g., burrs, and/or the like).

In some embodiments, a message 650 or other alert may be generated to alert a user to an invalid feature of the model configuration, for instance, providing information to indicate the source of the invalid feature.

In addition and/or in the alternative, indicators 625a-d may be configured to indicate a valid implant configuration and/or one or more features of a valid implant configuration). For example, indicators 625a-d may highlight compliant features (for instance, parameters, performance metrics, and/or the like), for example, using color, shading, borders, and/or the like.

Included herein are one or more logic flows representative of exemplary methodologies for performing novel features of the disclosed architecture. While, for purposes of simplicity of explanation, the one or more methodologies shown herein are shown and described as a series of acts, those skilled in the art will understand and appreciate that the methodologies are not limited by the order of acts. Some acts may, in accordance therewith, occur in a different order and/or concurrently with other acts from that shown and described herein. For example, those skilled in the art will understand and appreciate that a methodology could alternatively be represented as a series of interrelated states or events, such as in a state diagram. Moreover, not all acts illustrated in a methodology may be required for a novel implementation.

A logic flow may be implemented in software, firmware, hardware, or any combination thereof. In software and firmware embodiments, a logic flow may be implemented by computer executable instructions stored on a non-transitory computer readable medium or machine readable medium. The embodiments are not limited in this context.

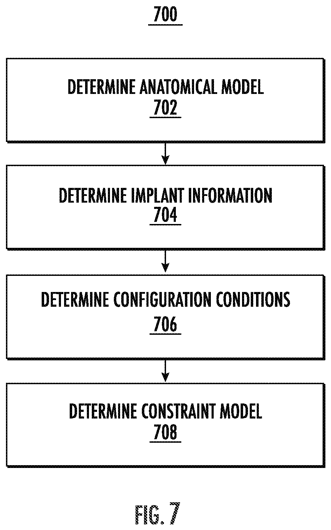

**FIG. 7** illustrates an embodiment of a logic flow 700. Logic flow 700 may be representative of some or all of the operations executed by one or more embodiments described herein, such as surgery system 250 and/or components thereof (for instance, computing device 210). In some embodiments, logic flow 700 may be representative of some or all of the operations of a surgical planning process, such as generating a constraint model, according to some embodiments.

At block 702, logic flow 700 may determine an anatomical model 702. For example, computer-assisted surgery application 260 may determine anatomical models 246, which may include virtual models of patient anatomy associated with a surgical procedure being performed via surgical system 230. In some embodiments, the virtual models may include mathematical models, graphical models, feature maps, combinations thereof, and/or the like configured to virtually represent patient anatomy within a computing system. In general, anatomical models according to some embodiments may be generated using various processes, including, without limitation, traditional probe painting, 3D imaging mapped with references, visual edge detection, combinations thereof, and/or the like.

Anatomical models may be determined based on physical information of the patient and may be generated via surgery system 230 and/or accessed from a data source, such as data sources 230a-n and/or anatomical models 246 stored in memory unit 240.

Logic flow 700 may determine implant information at block 704. For example, surgical system may determine or access implant information 244 may include information associated with an implant associated with a surgical procedure being performed via surgical system 230. In general, implant information 244 may include information required for modeling an implant and/or determining a configuration of an implant on or within patient anatomy. For example, implant information 244 for a femoral implant component may include a length and diameter of an implant stem, coupler information, and/or the like. Implant information 244 may be input by a user, accessed from a database, and/or determined based on manufacturer information.

At block 706, logic flow 706 may determine configuration conditions. For example, surgical system 230 may determine one or more conditions or requirements for installing the implant into the patient. Non-limiting examples of implant placement conditions may include, without limitation, fitting on or within anatomy (for instance, an implant stem of a tibial component fitting within the IM canal of the tibia), performance criteria or goals (for instance, placement of the implant may be required to meet certain performance metrics, such as range of motion, gait, deformities, and/or the like), placement criteria or goals (for instance, gap placement criteria, varus/valgus angle criteria, and/or the like), combinations thereof, and/or the like. In general, an implant condition is any parameter, setting, or other information, that may impact the validity of installing an implant on or within patient anatomy. For example, a user may specify that the installed implant have a varus/valgus angle of X. In another example, a healthcare facility may require that knee implants be installed with a performance metric meeting or exceeding a threshold. The implant conditions may specify the boundaries of a proper installation of an implant.

In some embodiments, the implant conditions may include variances or thresholds for invalid configurations. For example, an implant cannot be installed that violates actual physical dimensions of a joint (for instance, protruding into the wall of the IM canal). However, certain other conditions may be violated, such as a varus/valgus angle requirement or a performance metric requirement. Accordingly, in various embodiments, a user may specify (for instance, as a parameter 340 of UI screen 305) variances, tolerances, thresholds, and/or the like for one or more features of installation of an implant on or within patient anatomy. For example, a user may specify that a certain performance metric may be outside (below and/or above) a value by a threshold percent. In another example, a user may specify that gap tolerances may be outside (below and/or above) of specified values. In a further example, a user may specify that an IM stem may fit a threshold distance outside of a specified depth requirement.

At block 708, logic flow 700 may determine a constraint model. For example, surgical system 230 may generate a constraint model 248 formed from or otherwise associated with a constraint space in the form of a virtual representation of valid placement information for an implant within an anatomical model. For example, an anatomical model 246 may include a portion of a femur configured to receive a femur component of an implant. A corresponding constraint model 248 may be generated to represent, indicate, determine, or otherwise provide valid (or alternatively, invalid) placement locations, options, parameters, and/or the like

In some embodiments, for example, surgical system 230, for instance, via computer-assisted surgery logic 222, anatomical model logic 224, and/or a computer-assisted surgery application 260, may analyze all of the physical information associated with the model (for instance, IM canal volume, length, and/or the like) and physical information of an implant (for instance, stem dimensions, stem configurations (for instance, offset angles), and/or the like) in combination with implant conditions (for instance, performance metrics, parameter settings) and determine possible configurations that meet all requirements within the available space of the patient anatomy.

For example, computer-assisted surgery application 260 may determine a plurality of possible configurations for an IM stem within an IM canal of a patient based on the dimensions of the IM stem and the IM canal. Computer-assisted surgery application 260 may take implant conditions (for instance, performance metrics, parameter settings) and narrow down the possible configurations to determine one or more constraint models that are valid for the IM stem and IM canal physical dimensions in combination with the implant conditions to generate one or more constraint spaces or models. For example, a first constraint model may include a constraint space where an IM stem will fit within an IM canal at a varus/valgus angle of X degrees. In another example, a constraint space where an IM stem will fit within an IM canal at a varus/valgus angle of X degrees with a performance metric meeting a specified gap calculation goal.

In some embodiments, computational models, such as machine learning (ML), neural network (NN), and/or other artificial intelligence (AI) models may be trained to determine constraint models based on input information. In various embodiments, the computational models may be trained based on information associated with a population of patients with the same or similar surgical procedures, implants, anatomy, and/or the like. For example, a NN may be trained to receive input in the form of an anatomical model, an implant, and implant conditions and determine one or more constraint models, for instance, based on outcomes of a population of patients.

Although FIG. 7 depicts the determination of a constraint space including determining implant information and/or implant conditions, embodiments are not so limited. For example, in some embodiments, constraint spaces 248 may be generated based only on the physical anatomy of the corresponding patient anatomy (for instance, the dimensions of the IM canal of a patient). For instance, a constraint space may be based only on the physical volume and dimensions of the IM canal. In other embodiments, constraint spaces 248 may be generated based on the corresponding patient anatomy and implant information (for instance, dimensions, parameters, and/or the like). In various embodiments, constraint spaces 248 may be generated based on the corresponding patient anatomy, implant information, and implant conditions (for instance, parameter requirements (for example, a varus/valgus angle between X degrees and Y degrees), performance requirements, and/or the like).

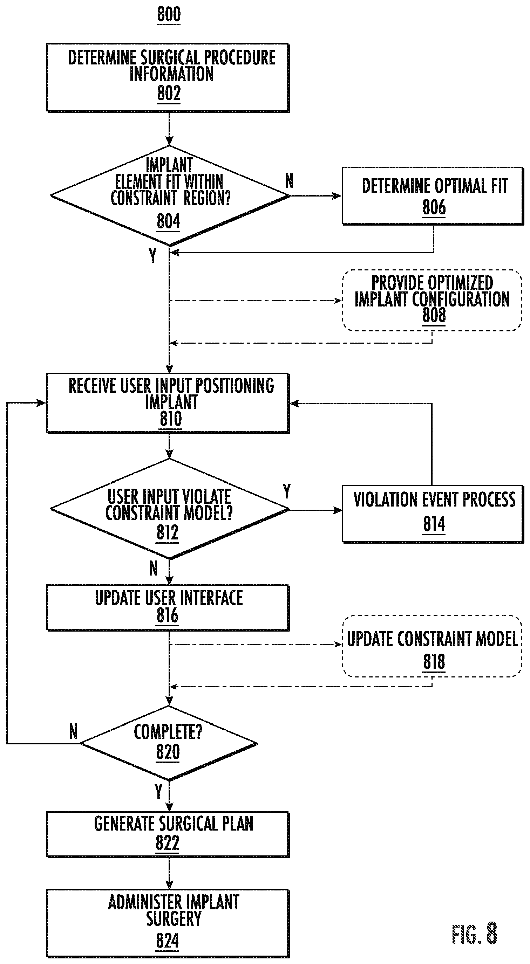

**FIG. 8** illustrates an embodiment of a logic flow 800. Logic flow 800 may be representative of some or all of the operations executed by one or more embodiments described herein, such as surgery system 250 and/or components thereof (for instance, computing device 210). In some embodiments, logic flow 800 may be representative of some or all of the operations of a surgical planning process according to some embodiments.

At block 802, logic flow 800 may determine surgical procedure information. For example, surgical system 230 may determine an anatomical model 310, a constraint model 314, and information for an implant 320.

Logic flow 800 may determine whether the implant my physically fit within a constraint region at block 804. If the implant cannot physically fit within the constraint region, logic flow 800 may determine an optimal fit at block 806. For example, if a placement of the implant violates the canal, a UI control or other function may trigger a best-fit (or other optimization) process that may move the implant to a closest position to the current location that doesn't violate the canal. In one illustrative and non-limiting example, a best-fit optimization process may be implemented via a breadth-first search through a degree-of-freedom (DOF) (for example, a 6-DOF) space within a pre-set distance (for instance, translation and/or rotation).

In some embodiments, logic flow 800 may provide an optimized implant configuration at block 808. For example, surgical planning processes may include an optimization process to determine an implant position and modular parameterization that maximally achieves the joint performance goals of the surgeon. An illustrative and non-limiting example of an optimization process may include a configuration space search which includes a heuristic cost function. For example, an implant position/joint offset parameterization could be selected that minimizes the calculated differences between flexion and extension gaps in the medial condyle, while maximizing the stem length width and length that fits in the canal volume, and further achieves a maximal centralization of the femoral implant between the femoral epicondyles.

Logic flow 800 may receive user input positioning implant at block 810. For example, with reference to FIGS. 3A-3C, a user may move implant 320 via an input device or function object 332a-n. In another example, a user may input a parameter and/or performance metric value.

At block 812, logic flow 800 may determine whether the user input has violated the constraint model. If the input violates the constraint model, logic flow 800 may activate a violation event process. For example, in some embodiments, a violation event process may operate to handle input that violates a constraint model. In various embodiments, a violation event process may prevent user input that violates a constraint model, for example, by preventing motion input moving the implant into or setting a parameter that causes a violating position. In another example, a violation event process may prevent input, for example, by limiting or blocking certain input, that may violate a constraint model (for instance, limiting a varus/valgus parameter value to be within a specified range). In an additional example, a violation event process may provide various indicators (see, for example, 371 and 372 of FIG. 3B) to visually communicate a violation status and/or particular violations of a constraint space.

If the input does not violate the constraint model, logic flow 800 may activate a placement process, placing or configuring the implant in association with the anatomical model according with the input.

At block 818, logic flow 800 may update a constraint model. For example, if the user input has changed the information used to generate the constraint model, such as an implant parameter or a required performance metric, the constraint model may be updated to reflect this change.

Logic flow 800 may determine whether the implant planning process is complete at block 820. If the implant planning process is complete, logic flow may generate a surgical plan 822 and/or administer the implant surgery 824 according to various embodiments described in the present disclosure.

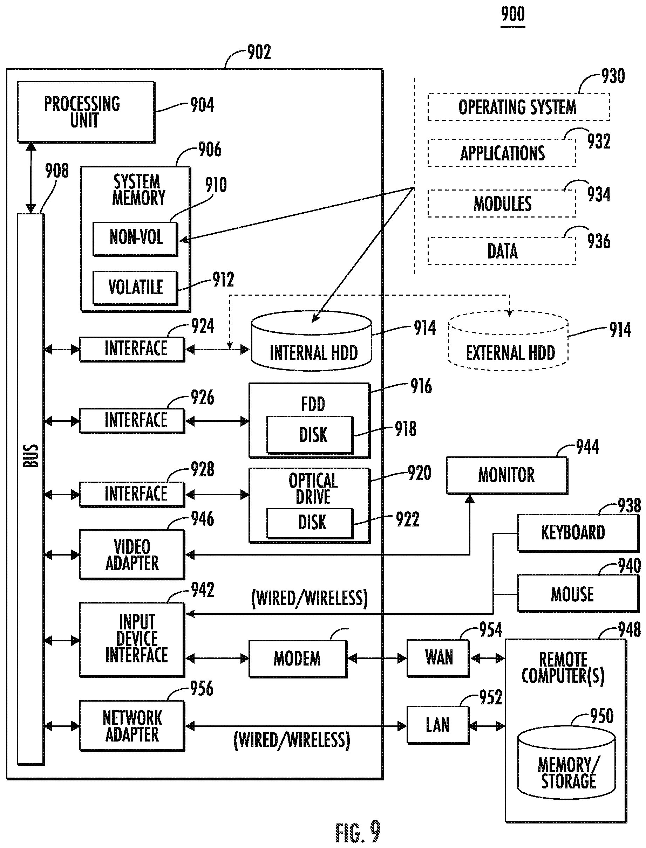

**FIG. 9** illustrates an embodiment of an exemplary computing architecture 900 suitable for implementing various embodiments as previously described. In various embodiments, the computing architecture 900 may comprise or be implemented as part of an electronic device. In some embodiments, the computing architecture 900 may be representative, for example, of computing device 210. The embodiments are not limited in this context.

As used in this application, the terms "system" and "component" and "module" are intended to refer to a computer-related entity, either hardware, a combination of hardware and software, software, or software in execution, examples of which are provided by the exemplary computing architecture 900. For example, a component can be, but is not limited to being, a process running on a processor, a processor, a hard disk drive, multiple storage drives (of optical and/or magnetic storage medium), an object, an executable, a thread of execution, a program, and/or a computer. By way of illustration, both an application running on a server and the server can be a component. One or more components can reside within a process and/or thread of execution, and a component can be localized on one computer and/or distributed between two or more computers. Further, components may be communicatively coupled to each other by various types of communications media to coordinate operations. The coordination may involve the uni-directional or bi-directional exchange of information. For instance, the components may communicate information in the form of signals communicated over the communications media. The information can be implemented as signals allocated to various signal lines. In such allocations, each message is a signal. Further embodiments, however, may alternatively employ data messages. Such data messages may be sent across various connections. Exemplary connections include parallel interfaces, serial interfaces, and bus interfaces.

The computing architecture 900 includes various common computing elements, such as one or more processors, multi-core processors, co-processors, memory units, chipsets, controllers, peripherals, interfaces, oscillators, timing devices, video cards, audio cards, multimedia input/output (I/O) components, power supplies, and so forth. The embodiments, however, are not limited to implementation by the computing architecture 900.

As shown in FIG. 9, the computing architecture 900 comprises a processing unit 904, a system memory 906 and a system bus 908. The processing unit 904 may be a commercially available processor and may include dual microprocessors, multi-core processors, and other multi-processor architectures.

The system bus 908 provides an interface for system components including, but not limited to, the system memory 906 to the processing unit 904. The system bus 908 can be any of several types of bus structure that may further interconnect to a memory bus (with or without a memory controller), a peripheral bus, and a local bus using any of a variety of commercially available bus architectures. Interface adapters may connect to the system bus 908 via a slot architecture. Example slot architectures may include without limitation Accelerated Graphics Port (AGP), Card Bus, (Extended) Industry Standard Architecture ((E)ISA), Micro Channel Architecture (MCA), NuBus, Peripheral Component Interconnect (Extended) (PCI(X)), PCI Express, Personal Computer Memory Card International Association (PCMCIA), and the like.

The system memory 906 may include various types of computer-readable storage media in the form of one or more higher speed memory units, such as read-only memory (ROM), random-access memory (RAM), dynamic RAM (DRAM), Double-Data-Rate DRAM (DDRAM), synchronous DRAM (SDRAM), static RAM (SRAM), programmable ROM (PROM), erasable programmable ROM (EPROM), electrically erasable programmable ROM (EEPROM), flash memory, polymer memory such as ferroelectric polymer memory, ovonic memory, phase change or ferroelectric memory, silicon-oxide-nitride-oxide-silicon (SONOS) memory, magnetic or optical cards, an array of devices such as Redundant Array of Independent Disks (RAID) drives, solid state memory devices (e.g., USB memory, solid state drives (SSD) and any other type of storage media suitable for storing information. In the illustrated embodiment shown in FIG. 9, the system memory 906 can include non-volatile memory 910 and/or volatile memory 912. A basic input/output system (BIOS) can be stored in the non-volatile memory 910.

The computer 902 may include various types of computer-readable storage media in the form of one or more lower speed memory units, including an internal (or external) hard disk drive (HDD) 914, a magnetic floppy disk drive (FDD) 916 to read from or write to a removable magnetic disk 911, and an optical disk drive 920 to read from or write to a removable optical disk 922 (e.g., a CD-ROM or DVD). The HDD 914, FDD 916 and optical disk drive 920 can be connected to the system bus 908 by a HDD interface 924, an FDD interface 926 and an optical drive interface 928, respectively. The HDD interface 924 for external drive implementations can include at least one or both of Universal Serial Bus (USB) and IEEE 1114 interface technologies.

The drives and associated computer-readable media provide volatile and/or nonvolatile storage of data, data structures, computer-executable instructions, and so forth. For example, a number of program modules can be stored in the drives and memory units 910, 912, including an operating system 930, one or more application programs 932, other program modules 934, and program data 936. In one embodiment, the one or more application programs 932, other program modules 934, and program data 936 can include, for example, the various applications and/or components of computing device 110.

A user can enter commands and information into the computer 902 through one or more wired/wireless input devices, for example, a keyboard 938 and a pointing device, such as a mouse 940. These and other input devices are often connected to the processing unit 904 through an input device interface 942 that is coupled to the system bus 908, but can be connected by other interfaces.

A monitor 944 or other type of display device is also connected to the system bus 908 via an interface, such as a video adaptor 946. The monitor 944 may be internal or external to the computer 902. In addition to the monitor 944, a computer typically includes other peripheral output devices, such as speakers, printers, and so forth.

The computer 902 may operate in a networked environment using logical connections via wired and/or wireless communications to one or more remote computers, such as a remote computer 948. The remote computer 948 can be a workstation, a server computer, a router, a personal computer, portable computer, microprocessor-based entertainment appliance, a peer device or other common network node, and typically includes many or all of the elements described relative to the computer 902, although, for purposes of brevity, only a memory/storage device 950 is illustrated. The logical connections depicted include wired/wireless connectivity to a local area network (LAN) 952 and/or larger networks, for example, a wide area network (WAN) 954. Such LAN and WAN networking environments are commonplace in offices and companies, and facilitate enterprise-wide computer networks, such as intranets, all of which may connect to a global communications network, for example, the Internet.

The computer 902 is operable to communicate with wired and wireless devices or entities using the IEEE 802 family of standards, such as wireless devices operatively disposed in wireless communication (e.g., IEEE 802.16 over-the-air modulation techniques). This includes at least Wi-Fi (or Wireless Fidelity), WiMax, and Bluetooth^{™} wireless technologies, among others. Thus, the communication can be a predefined structure as with a conventional network or simply an ad hoc communication between at least two devices. Wi-Fi networks use radio technologies called IEEE 802.11x (a, b, g, n, etc.) to provide secure, reliable, fast wireless connectivity. A Wi-Fi network can be used to connect computers to each other, to the Internet, and to wire networks (which use IEEE 802.3-related media and functions).

While the present disclosure refers to certain embodiments, numerous modifications, alterations, and changes to the described embodiments are possible without departing from the sphere and scope of the present disclosure, as defined in the appended claim(s). Accordingly, it is intended that the present disclosure not be limited to the described embodiments, but that it has the full scope defined by the language of the following claims, and equivalents thereof. The discussion of any embodiment is meant only to be explanatory and is not intended to suggest that the scope of the disclosure, including the claims, is limited to these embodiments. In other words, while illustrative embodiments of the disclosure have been described in detail herein, it is to be understood that the inventive concepts may be otherwise variously embodied and employed, and that the appended claims are intended to be construed to include such variations, except as limited by the prior art.

Directional terms such as top, bottom, superior, inferior, medial, lateral, anterior, posterior, proximal, distal, upper, lower, upward, downward, left, right, longitudinal, front, back, above, below, vertical, horizontal, radial, axial, clockwise, and counterclockwise) and the like may have been used herein. Such directional references are only used for identification purposes to aid the reader's understanding of the present disclosure. For example, the term "distal" may refer to the end farthest away from the medical professional/operator when introducing a device into a patient, while the term "proximal" may refer to the end closest to the medical professional when introducing a device into a patient. Such directional references do not necessarily create limitations, particularly as to the position, orientation, or use of this disclosure. As such, directional references should not be limited to specific coordinate orientations, distances, or sizes, but are used to describe relative positions referencing particular embodiments. Such terms are not generally limiting to the scope of the claims made herein. Any embodiment or feature of any section, portion, or any other component shown or particularly described in relation to various embodiments of similar sections, portions, or components herein may be interchangeably applied to any other similar embodiment or feature shown or described herein.

While the present disclosure refers to certain embodiments, numerous modifications, alterations, and changes to the described embodiments are possible without departing from the sphere and scope of the present disclosure, as defined in the appended claim(s). Accordingly, it is intended that the present disclosure not be limited to the described embodiments. Rather these embodiments should be considered as illustrative and not restrictive in character. The present disclosure should be given the full scope defined by the language of the following claims, and equivalents thereof. Unless otherwise defined, all technical terms used herein have the same meaning as commonly understood by one of ordinary skill in the art to which the disclosure belongs.

The foregoing description has broad application. The discussion of any embodiment is meant only to be explanatory and is not intended to suggest that the scope of the disclosure, including the claims, is limited to these embodiments. In other words, while illustrative embodiments of the disclosure have been described in detail herein, it is to be understood that the inventive concepts may be otherwise variously embodied and employed, and that the appended claims are intended to be construed to include such variations, except as limited by the prior art.

It should be understood that, as described herein, an "embodiment" (such as illustrated in the accompanying Figures) may refer to an illustrative representation of an environment or article or component in which a disclosed concept or feature may be provided or embodied, or to the representation of a manner in which just the concept or feature may be provided or embodied. However, such illustrated embodiments are to be understood as examples (unless otherwise stated), and other manners of embodying the described concepts or features, such as may be understood by one of ordinary skill in the art upon learning the concepts or features from the present disclosure, are within the scope of the disclosure. Furthermore, references to "one embodiment" of the present disclosure are not intended to be interpreted as excluding the existence of additional embodiments that also incorporate the recited features.

In addition, it will be appreciated that while the Figures may show one or more embodiments of concepts or features together in a single embodiment of an environment, article, or component incorporating such concepts or features, such concepts or features are to be understood (unless otherwise specified) as independent of and separate from one another and are shown together for the sake of convenience and without intent to limit to being present or used together. For instance, features illustrated or described as part of one embodiment can be used separately, or with another embodiment to yield a still further embodiment. Thus, it is intended that the present subject matter covers such modifications and variations as come within the scope of the appended claims and their equivalents.

As used herein, an element or step recited in the singular and proceeded with the word "a" or "an" should be understood as not excluding plural elements or steps, unless such exclusion is explicitly recited. It will be further understood that the terms "comprises" and/or "comprising," or "includes" and/or "including" when used herein, specify the presence of stated features, regions, steps, elements and/or components, but do not preclude the presence or addition of one or more other features, regions, integers, steps, operations, elements, components and/or groups thereof.

The phrases "at least one", "one or more", and "and/or", as used herein, are openended expressions that are both conjunctive and disjunctive in operation. The terms "a" (or "an"), "one or more" and "at least one" can be used interchangeably herein.

Connection references (e.g., engaged, attached, coupled, connected, and joined) are to be construed broadly and may include intermediate members between a collection of elements and relative to movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another. The drawings are for purposes of illustration only and the dimensions, positions, order and relative to sizes reflected in the drawings attached hereto may vary.

The foregoing discussion has been presented for purposes of illustration and description and is not intended to limit the disclosure to the form or forms disclosed herein. For example, various features of the disclosure are grouped together in one or more embodiments or configurations for the purpose of streamlining the disclosure. However, it should be understood that various features of the certain embodiments or configurations of the disclosure may be combined in alternate embodiments or configurations. Moreover, the following claims are hereby incorporated into this Detailed Description by this reference, with each claim standing on its own as a separate embodiment of the present disclosure.

## Claims

1. A surgical system (100, 230), comprising:
at least one computing device (210), comprising:
processing circuitry (220); and
a memory (240) coupled to the processing circuitry, the memory comprising instructions that, when executed by the processing circuitry, causes the processing circuitry to:
determine a constraint model (248, 314, 454, 614) associated with an anatomical model (246, 310, 610, 702), the constraint model configured to model at least one valid configuration of an implant model in association with the anatomical model, wherein the anatomical model comprises at least one model interface (312) for engaging an implant interface (322) of the implant model, the constraint model configured to indicate at least one valid configuration of the implant interface in association with the model interface,
receive placement input indicating placement of the implant in association with the anatomical model, and
perform, based on the placement input one of:
a violation process based on a violation event responsive to the placement input violating the constraint model, the violation process operative to prevent placement input moving the implant into a position that violates the constraint model, or
a placement process positioning the implant according to the placement input responsive to the placement input not violating the constraint model, **characterised in that** the model interface comprises an intramedullary (IM) canal interface, the implant interface comprising an IM stem configured to engage the IM canal, wherein the constraint model comprises a constraint space defined, at least partially, by a volume of the IM canal.

2. The surgical system of claim 1, wherein the instructions, when executed by the processing circuitry, cause the processing circuitry to generate at least one indicator (371, 625) indicating a violation responsive to the violation event.

3. The surgical system of claim 2, wherein the at least one indicator comprises at least one of an overlay, a color, a pattern, an alert, an alarm, a message, or a tactile signal.

4. The surgical system of any of claims 1-3, wherein the violation event process is operative to prevent user input that violates the constraint model via limiting parameter values that violate the constraint model.

5. The surgical system of any of claims 1-4, wherein movement of the model is bound within a region defined by the constraint model to prevent model configurations that violate the constraint model.

6. The surgical system of any of claims 1-5, wherein the instructions, when executed by the processing circuitry, cause the processing circuitry to generate a surgical plan (250) determined based on a configuration of the implant model within the constraint model.

7. The surgical system of any of claims 1-6, wherein the constraint model is configured for an arthroplasty surgical procedure.

8. The surgical system of claim 7, wherein the anatomical model comprises at least a portion of a distal femur configured to receive a femoral component knee implant device.

9. The surgical system of claim 7, wherein the anatomical model comprises at least a portion of a proximal tibia configured to receive a tibial knee implant device.

10. The surgical system of any of claims 1-9, wherein the at least one valid configuration based on at least one implant placement condition comprises at least one of an anatomical fit or a performance criterion of an implant.

11. The surgical system of any of claims 1-10, wherein the constraint model is configured to model at least one degree-of-freedom of an implant, the at least one degree-of-freedom comprising at least one of a varus/valgus angle, a flexion/extension position, a medial/lateral position, or an anterior/posterior position

12. The surgical system of any of claims 1-11, wherein the constraint model is generated to correspond to surgically-prepared patient anatomy.

## Patentansprüche

1. Chirurgisches System (100, 230), umfassend:
mindestens eine Computervorrichtung (210), die Folgendes umfasst:
eine Verarbeitungsschaltungsanordnung (220); und
einen Speicher (240), der mit der Verarbeitungsschaltungsanordnung gekoppelt ist, wobei der Speicher Anweisungen umfasst, die, wenn sie von der Verarbeitungsschaltungsanordnung ausgeführt werden, die Verarbeitungsschaltungsanordnung zu Folgendem veranlassen:
Bestimmen eines Beschränkungsmodells (248, 314, 454, 614), das mit einem anatomischen Modell (246, 310, 610, 702) assoziiert ist, wobei das Beschränkungsmodell dazu ausgelegt ist, mindestens eine gültige Konfiguration eines Implantatmodells in Verbindung mit dem anatomischen Modell zu modellieren, wobei das anatomische Modell mindestens eine Modellschnittstelle (312) zum Eingreifen in eine Implantatschnittstelle (322) des Implantatmodells umfasst, wobei das Beschränkungsmodell dazu ausgelegt ist, mindestens eine gültige Konfiguration der Implantatschnittstelle in Verbindung mit der Modellschnittstelle anzugeben,
Empfangen einer Platzierungseingabe, die eine Platzierung des Implantats in Verbindung mit dem anatomischen Modell angibt, und
Durchführen eines von Folgenden basierend auf der Platzierungseingabe:
einen Verletzungsprozess basierend auf einem Verletzungsereignis in Reaktion darauf, dass die Platzierungseingabe das Beschränkungsmodell verletzt, wobei der Verletzungsprozess wirksam ist, um zu verhindern, dass die Platzierungseingabe das Implantat in eine Position bewegt, die das Beschränkungsmodell verletzt, oder
einen Platzierungsprozess, der das Implantat gemäß der Platzierungseingabe in Reaktion darauf positioniert, dass die Platzierungseingabe das Beschränkungsmodell nicht verletzt, **dadurch gekennzeichnet, dass** die Modellschnittstelle eine Intramedullärkanalschnittstelle (IM-Kanalschnittstelle) umfasst, wobei die Implantatschnittstelle einen IM-Schaft umfasst, der dazu ausgelegt ist, in den IM-Kanal einzugreifen, wobei das Beschränkungsmodell einen Beschränkungsraum umfasst, der zumindest teilweise durch ein Volumen des IM-Kanals definiert ist.

2. Chirurgisches System nach Anspruch 1, wobei die Anweisungen, wenn sie von der Verarbeitungsschaltungsanordnung ausgeführt werden, die Verarbeitungsschaltungsanordnung veranlassen, mindestens einen Indikator (371, 625) zu erstellen, der in Reaktion auf das Verletzungsereignis eine Verletzung angibt.

3. Chirurgisches System nach Anspruch 2, wobei der mindestens eine Indikator mindestens eines von einer Überlagerung, einer Farbe, einem Muster, einer Warnung, einem Alarm, einer Nachricht oder einem taktilen Signal umfasst.

4. Chirurgisches System nach einem der Ansprüche 1-3, wobei der Verletzungsereignisprozess wirksam ist, um durch Limitieren von Parameterwerten, die das Beschränkungsmodell verletzen, eine Benutzereingabe zu verhindern, die das Beschränkungsmodell verletzt.

5. Chirurgisches System nach einem der Ansprüche 1-4, wobei die Bewegung des Modells innerhalb eines Bereichs begrenzt ist, der durch das Beschränkungsmodell definiert ist, um Modellkonfigurationen zu verhindern, die das Beschränkungsmodell verletzen.

6. Chirurgisches System nach einem der Ansprüche 1-5, wobei die Anweisungen, wenn sie von der Verarbeitungsschaltungsanordnung ausgeführt werden, die Verarbeitungsschaltungsanordnung veranlassen, einen chirurgischen Plan (250) zu erstellen, der basierend auf einer Konfiguration des Implantatmodells innerhalb des Beschränkungsmodells bestimmt wird.

7. Chirurgisches System nach einem der Ansprüche 1-6, wobei das Beschränkungsmodell für einen arthroplastischchirurgischen Eingriff ausgelegt ist.

8. Chirurgisches System nach Anspruch 7, wobei das anatomische Modell mindestens einen Abschnitt eines distalen Femurs umfasst, der dazu ausgelegt ist, eine Knieimplantatvorrichtung mit Femurkomponente aufzunehmen.

9. Chirurgisches System nach Anspruch 7, wobei das anatomische Modell mindestens einen Abschnitt einer proximalen Tibia umfasst, der dazu ausgelegt ist, eine Tibia-Knieimplantatvorrichtung aufzunehmen.

10. Chirurgisches System nach einem der Ansprüche 1-9, wobei die mindestens eine gültige Konfiguration basierend auf mindestens einer Implantatplatzierungsbedingung mindestens eines von einer anatomischen Passform oder einem Leistungskriterium eines Implantats umfasst.

11. Chirurgisches System nach einem der Ansprüche 1-10, wobei das Beschränkungsmodell dazu ausgelegt ist, mindestens einen Freiheitsgrad eines Implantats zu modellieren, wobei der mindestens eine Freiheitsgrad mindestens eines von einem Varus-/Valguswinkel, einer Flexions-/Extensionsposition, einer medialen/lateralen Position oder einer anterioren/posterioren Position umfasst.

12. Chirurgisches System nach einem der Ansprüche 1-11, wobei das Beschränkungsmodell so erstellt wird, dass es einer chirurgisch vorbereiteten Patientenanatomie entspricht.

## Revendications

1. Système chirurgical (100, 230), comprenant :
au moins un dispositif informatique (210), comprenant :
un circuit de traitement (220) ; et
une mémoire (240) couplée au circuit de traitement, la mémoire comprenant des instructions qui, lorsqu'elles sont exécutées par le circuit de traitement, amènent le circuit de traitement à :
déterminer un modèle de contrainte (248, 314, 454, 614) associé à un modèle anatomique (246, 310, 610, 702), le modèle de contrainte étant configuré pour modéliser au moins une configuration valide d'un modèle d'implant en association avec le modèle anatomique, le modèle anatomique comprenant au moins une interface de modèle (312) pour venir en prise avec une interface d'implant (322) du modèle d'implant, le modèle de contrainte étant configuré pour indiquer au moins une configuration valide de l'interface d'implant en association avec l'interface de modèle,
recevoir une entrée de placement indiquant le placement de l'implant en association avec le modèle anatomique, et
effectuer, sur la base de l'entrée de placement, l'une des opérations suivantes :
un processus de violation basé sur un événement de violation en réponse à l'entrée de placement violant le modèle de contrainte, le processus de violation étant opérationnel pour empêcher l'entrée de placement de déplacer l'implant dans une position qui viole le modèle de contrainte, ou
un processus de placement positionnant l'implant selon l'entrée de placement en réponse à l'entrée de placement ne violant pas le modèle de contrainte, **caractérisé en ce que** l'interface de modèle comprend une interface de canal intramédullaire (IM), l'interface d'implant comprenant une tige IM configurée pour venir en prise avec le canal IM, le modèle de contrainte comprenant un espace de contrainte défini, au moins partiellement, par un volume du canal IM.

2. Système chirurgical selon la revendication 1, les instructions, lorsqu'elles sont exécutées par le circuit de traitement, amenant le circuit de traitement à générer au moins un indicateur (371, 625) indiquant une violation en réponse à l'événement de violation.

3. Système chirurgical selon la revendication 2, l'au moins un indicateur comprenant au moins l'un d'un recouvrement, d'une couleur, d'un motif, d'une alerte, d'une alarme, d'un message ou d'un signal tactile.

4. Système chirurgical selon l'une quelconque des revendications 1 à 3, le processus d'événement de violation étant opérationnel pour empêcher une entrée utilisateur qui viole le modèle de contrainte par l'intermédiaire de la limitation de valeurs de paramètres qui violent le modèle de contrainte.

5. Système chirurgical selon l'une quelconque des revendications 1 à 4, le mouvement du modèle étant limité à l'intérieur d'une région définie par le modèle de contrainte pour empêcher des configurations de modèle qui violent le modèle de contrainte.

6. Système chirurgical selon l'une quelconque des revendications 1 à 5, les instructions, lorsqu'elles sont exécutées par le circuit de traitement, amenant le circuit de traitement à générer un plan chirurgical (250) déterminé sur la base d'une configuration du modèle d'implant à l'intérieur du modèle de contrainte.

7. Système chirurgical selon l'une quelconque des revendications 1 à 6, le modèle de contrainte étant configuré pour une procédure chirurgicale d'arthroplastie.

8. Système chirurgical selon la revendication 7, le modèle anatomique comprenant au moins une partie d'un fémur distal configurée pour recevoir un dispositif d'implant de genou à composant fémoral.

9. Système chirurgical selon la revendication 7, le modèle anatomique comprenant au moins une partie d'un tibia proximal configurée pour recevoir un dispositif d'implant de genou tibial.

10. Système chirurgical selon l'une quelconque des revendications 1 à 9, l'au moins une configuration valide sur la base d'au moins une condition de placement d'implant comprenant au moins l'un d'un ajustement anatomique ou d'un critère de performance d'un implant.

11. Système chirurgical selon l'une quelconque des revendications 1 à 10, le modèle de contrainte étant configuré pour modéliser au moins un degré de liberté d'un implant, l'au moins un degré de liberté comprenant au moins l'un d'un angle varus/valgus, d'une position de flexion/extension, d'une position médiale/latérale ou d'une position antérieure/postérieure.

12. Système chirurgical selon l'une quelconque des revendications 1 à 11, le modèle de contrainte étant généré pour correspondre à l'anatomie de patient préparée chirurgicalement.
